# EUROPÄISCHE PATENTANMELDUNG

(11) **EP 1 164 126 A1**
(43) Veröffentlichungstag der Anmeldung: **19.12.2001**
(21) Anmeldenummer: 01114056.3
(22) Anmeldetag: 08.06.2001
(51) Int. Cl.: C07C 251/86, A01N 33/26, A01N 37/28

(54) **Salicylsäurehydrazid-Derivate, Verfahren und Zwischenprodukte zu ihrer Herstellung, sie enthaltende Mittel und ihre Verwendung zur Bekämpfung von Schadpilzen**

(30) Priorität: 16.06.2000 DE 10028978
(71) Anmelder: BASF AKTIENGESELLSCHAFT, 67056 Ludwigshafen (DE)
(72) Erfinder: Gypser, Andreas, Dr., 68159 Mannheim (DE); Grote, Thomas, Dr., 67157 Wachenheim (DE); Rheinheimer, Joachim, Dr., 67063 Ludwigshafen (DE); Rose, Ingo, Dr., 68159 Mannheim (DE); Schäfer, Peter, Dr., 67308 Ottersheim (DE); Cullmann, Oliver, Dr., 64646 Heppenheim (DE); Gewehr, Markus, Dr., 56288 Kastellaun (DE); Grammenos, Wassilios, Dr., 67063 Ludwigshafen (DE); Tormo i Blasco, Jordi, Dr., 67117 Limburgerhof (DE); Müller, Bernd, Dr., 67227 Frankenthal (DE); Sauter, Hubert, Dr., 68167 Mannheim (DE); Ammermann, Eberhard, Dr., 64646 Heppenheim (DE); Strathmann, Siegfried, Dr., 67117 Limburgerhof (DE); Lorenz, Gisela, Dr., 67434 Hambach (DE); Stierl, Reinhard, Dr., 67112 Mutterstadt (DE)

(57) **Zusammenfassung**

Salicylsäurehydrazid-Derivate der Formel I, 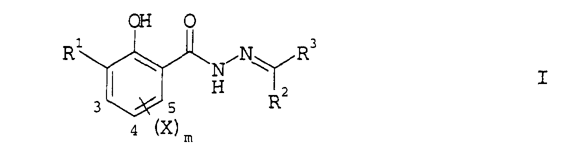 in der der Index und die Substituenten folgende Bedeutung haben:
- X: Halogen, NO₂, Cyano, Alkyl oder Alkoxy;
- m: 0, 1, 2 oder 3, wobei die Substituenten X verschieden sein können, wenn n größer als 1 ist;
- R¹: NO₂, NH₂ oder NH-CO-A;
A Wasserstoff, Alkyl, Alkoxy, NH₂, NHCH₃ oder N(CH₃)₂;
- R²: Wasserstoff, Cyano, Alkyl, Alkenyl, Alkinyl, Halogenalkyl, Alkoxy oder Alkylthio;
wobei die Kohlenwasserstoffreste unsubstituiert oder substituiert sein können
- R³: Phenyl, Naphthyl, Cycloalkyl, 5-gliedriges oder 6-gliedriges Hetaryl oder 5-gliedriges oder 6-gliedriges Heterocyclyl, enthaltend ein bis drei N-Atome und/oder ein O- oder S-Atom oder ein oder zwei O- und/oder S-Atome, wobei die Ringsysteme unsubstituiert oder substituiert sind;

Verfahren zur Herstellung dieser Verbindungen, sie enthaltende Mittel sowie ihre Verwendung zur Bekämpfung von Schadpilzen.

## Beschreibung

Die vorliegende Erfindung betrifft Salicylsäurehydrazid-Derivate der Formel I, 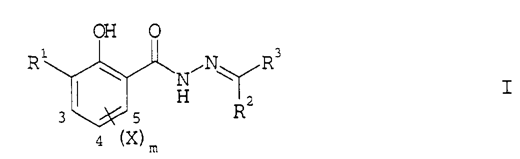 in der der Index und die Substituenten folgende Bedeutung haben:
- X: Halogen, NO₂, Cyano, C₁-C₄-Alkyl oder C₁-C₄-Alkoxy;
- m: 0, 1, 2 oder 3, wobei die Substituenten X verschieden sein können, wenn n größer als 1 ist;
- R¹: NO₂, NH₂ oder NH-CO-A;
A Wasserstoff, C₁-C₄-Alkyl, C₁-C₄-Alkoxy, NH₂, NHCH₃ oder N(CH₃)₂;
- R²: Wasserstoff, Cyano, C₁-C₆-Alkyl, C₂-C₆-Alkenyl, C₂-C₆-Alkinyl, C₁-C₆-Halogenalkyl, C₁-C₆-Alkoxy oder C₁-C₆-Alkylthio;
wobei die Kohlenwasserstoffreste unsubstituiert oder partiell oder vollständig halogeniert sind oder eine bis drei Gruppen R^{a} tragen können
R^{a} Halogen, Cyano, Nitro, Hydroxy, C₁-C₆-Alkyl, C₁-C₆-Halo-genalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino,
Di-C₁-C₆-alkylamino, C₂-C₆-Alkenyl, C₃-C₆-Alkenyloxy, C₃-C₆-Alkinyloxy oder C₁-C₄-Alkylendioxy, welches halogeniert sein kann,
- R³: Phenyl, Naphthyl, C₃-C₁₀-Cycloalkyl, 5-gliedriges oder 6-gliedriges Hetaryl oder 5-gliedriges oder 6-gliedriges Heterocyclyl, enthaltend ein bis drei N-Atome und/oder ein O- oder S-Atom oder ein oder zwei O- und/oder S-Atome, wobei die Ringsysteme unsubstituiert oder substituiert sind durch einen bis drei Reste R^{b}:
R^{b} Cyano, Nitro, Amino, Aminocarbonyl, Aminothiocarbonyl, Halogen, Hydroxy, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Al-kylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Al-kenyloxy, Phenyl, Phenoxy, Benzyl, Benzyloxy, 5- oder 6-gliedriges Heterocyclyl, 5- oder 6-gliedriges Hetaryl, 5- oder 6-gliedriges Hetaryloxy, C(=NOR^{α})-OR^{β} oder OC(R^{α} )₂-C(R^{β})=NOR^{β},
wobei die cyclischen Reste ihrerseits unsubstituiert oder substituiert sind durch einen bis drei Reste R^{c}:
R^{c} Cyano, Nitro, Halogen, Hydroxy, Amino, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkenyl, Phenyl, Phenoxy, Phenylthio, Benzyl, Benzyloxy, 5- oder 6-gliedriges Heterocyclyl, 5- oder 6-gliedriges Hetaryl, 5- oder 6-gliedriges Hetaryloxy oder C(=NOR^{α})-OR^{β};
R^{α}, R^{β} Wasserstoff oder C₁-C₆-Alkyl.

Außerdem betrifft die Erfindung Verfahren zur Herstellung dieser Verbindungen, sie enthaltende Mittel sowie ihre Verwendung zur Bekämpfung von Schadpilzen.

Aus WO-A 97/08135, DE-A 197 10 609 und WO-A 99/27783 sind Acylamino-Salicylsäureamide zur Bekämpfung von Schadpilzen bekannt.

Deren Wirkung kann jedoch in vielen Fällen nicht befriedigen. Der vorliegenden Erfindung lagen Verbindungen mit verbesserter Wirkung als Aufgabe zugrunde.

Demgemäß wurden die eingangs definierten Verbindungen gefunden. Desweiteren wurden Verfahren zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus dem Stand der Technikb bekannten durch die Hydrazid-Gruppierung.

Die Verbindungen der Formel I weisen eine gegenüber den bekannten Verbindungen erhöhte Wirksamkeit gegen Schadpilze auf.

Verbindungen der Formel I können beispielsweise ausgehend von Hydraziden der Formel II hergestellt werden; dabei werden Hydrazide mit Carbonylverbindungen der Formel III kondensiert. Die Verbindungen der Formel IA werden auch als Zwischenprodukte zur Herstellung weiterer Verbindungen I verwendet.

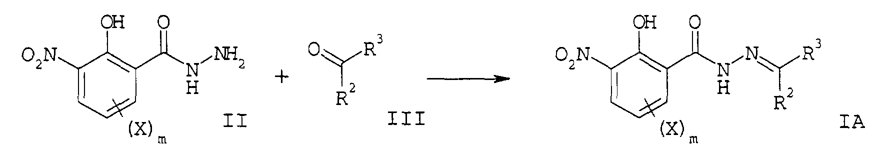

Diese Umsetzung erfolgt üblicherweise bei Temperaturen von 20°C bis 100°C, vorzugsweise 20°C bis 50°C, in einem inerten organischen Lösungsmittel in Gegenwart einer Säure [vgl. Indian J. Chem. B, Bd. 24, S. 979 (1985)].

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan und Tetrahydrofuran, Nitrile wie Acetonitril und Propionitril, Alkohole wie Methanol, Ethanol, n-Propanol, Isopropanol und n-Butanol, sowie Dimethylsulfoxid, Dimethylformamid und Dimethylacetamid,besonders bevorzugt Alkohole wie Methanol, Ethanol, n-Propanol, Isopropanol und n-Butanol. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure, Trifluoressigsäure, p-Toluolsulfonsäure, Benzolsulfonsäure und Camphersulfonsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, III in einem Überschuß bezogen auf II einzusetzen.

Die für die Herstellung der Verbindungen I benötigten Ausgangsstoffe II und III sind kommerziell erhältlich, in der Literatur bekannt [ZA 70/00662; Labdev, Part A, Bd. 11A(1-2), S. 35 (1973)] oder können gemäß der zitierten Literatur hergestellt werden.

Zur Herstellung von Verbindungen I, in denen R¹ NH₂ oder NH-CO-A bedeutet, werden Hydrazide der Formel IA zu Aminophenolverbindungen der Formel IB reduziert.

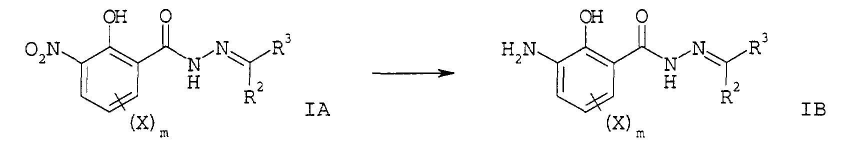

Die Reduktion der Nitrogruppe von IA kann unter allgemein üblichen Bedingungen durchgeführt werden, durch Reduktion mit Eisen, Zinn oder Zink in Gegenwart einer Säure oder durch enzymkatalysierte Reduktion [vgl. Houben-Weyl, Bd. IV/1c, 4. Aufl., S. 506, Thieme Verlag Stuttgart und New York (1980); ebd. Bd. IV/1d, 4. Aufl., S. 473 (1981); Heterocycles, Bd. 31, S. 2201 (1990)].

Bevorzugt erfolgt die Umsetzung mit Wasserstoff durch katalytische Hydrierung bei -20°C und + 180°C, vorzugsweise zwischen -5 und +40°C. Die Mindesttemperatur ist nur durch den Gefrierpunkt des verwendeten Lösungsmittels bestimmt. Üblicherweise wird bei einem Wasserstoffdruck hydriert, der zwischen Normaldruck und 30 bar Überdruck liegt. Normalerweise wird der Wasserstoff bei Normaldruck bzw. leicht erhöhtem Druck eingegast [vgl. WO-A 97/08135].

Als Katalysatoren bei der katalytischen Hydrierung werden handelsübliche Katalysatoren, die beispielsweise Platin, Platinoxid oder Palladium auf einem Träger enthalten, oder auch Raney-Nickel oder Raney-Cobalt, eingesetzt.

Die Verwendung von Platin- oder Palladiumkatalysatoren ist bevorzugt. Der Platin- bzw. Palladiumgehalt des Katalysators ist nicht kritisch und kann in weiten Grenzen variiert werden. Zweckmäßig ist ein Gehalt von 0,1 bis 15 Gew.-%, vorzugsweise von 0,5 bis 10 Gew.-%, bezogen auf das Trägermaterial. Die Menge des eingesetzten Platins oder Palladiums beträgt zwischen 0,001 und 10 Gew.-%, bevorzugt zwischen 0,01 und 0,1 Gew.-%, bezogen auf die Nitroverbindung. In der bevorzugten Ausführungsform wird als Trägermaterial Kohle verwendet. Andere nicht amphotere Träger, wie Graphit, BaSO₄ oder SiC sind ebenfalls geeignet.

Geeignete Verdünnungsmittel sind Ester wie Essigsäureethylester, Alkohole, wie Methanol, Ethanol, n-Propanol, n-, i-, sek- oder tert-Butanol, Ethandiol, Propan-1,2-diol, Ethoxyethnol, Methoxyethanol, Ether wie Diethylengylkolmonomethylether, Diethylenglykolmonoethylether, Wasser, wässrige Salzlösungen, wie beispielsweise Ammoniumchloridlösung, Säuren, wie beispielsweise Salzsäure oder Essigsäure; besonders bevorzugt sind Methanol, Ethanol und Wasser. Es können auch Gemische der genannten Verdünnungsmittel verwendet werden.

Die Säuren werden im allgemeinen in katalytischen Mengen eingesetzt, sie können aber auch äquimolar, im Überschuß oder gegebenenfalls als Lösungsmittel verwendet werden.

Zur Herstellung von Verbindungen I, in denen R¹ NH-CO-A bedeutet, werden Aminophenole der Formel IB als Zwischenprodukte verwendet.

Die Formylierung der Aminophenole der Formel IB erfolgt bevorzugt mit Ameisensäure. Es werden direkt die Verbindungen der Formel I, in der R¹ für NH-CO-H steht, erhalten; sie entsprechen der Formel I.1.

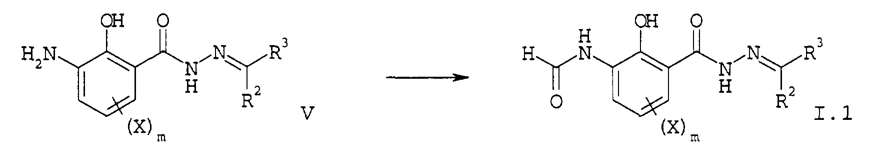

Diese Umsetzung erfolgt üblicherweise bei Temperaturen von 20°C bis 100°C, vorzugsweise 20°C bis 80°C, in einem inerten organischen Lösungsmittel in Gegenwart einer Säure.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure, Trifluoressigsäure, p-Toluolsulfonsäure, Benzolsulfonsäure und Camphersulfonsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, Ameisensäure in einem Überschuß bezogen auf V einzusetzen.

Aus den Verbindungen der Formel IB können die Verbindungen der Formel I, in denen R¹ für NH-CO-A bedeutet, wobei A für NH₂, NHCH₃, N(CH₃)₂ oder OCH₃ steht, erhalten werden.

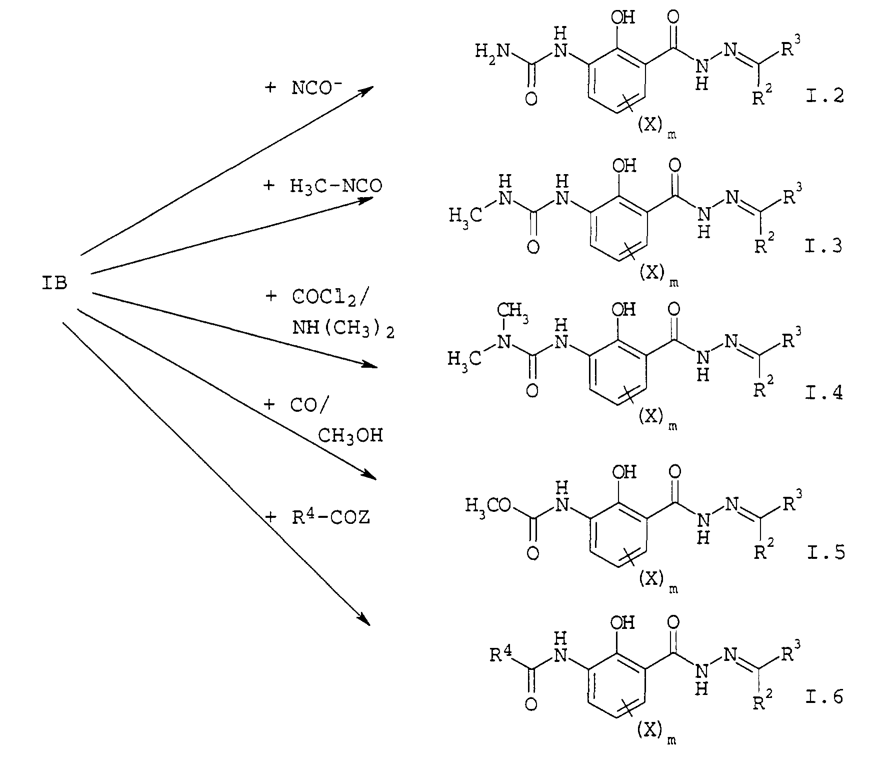

Die Derivatisierungen der Verbindungen der Formel IB zu den Verbindungen der Formel I.2 bis I.6 erfolgen nach aus folgenden Dokumenten bekannten Methoden, deren Offenbarung hiermit einbezogen ist: J. Med. Chem., Bd. 32, S. 990-997 (1989); J. Org. Chem., Bd. 26, S. 5238 (1961); J. Chem. Res. Synop, 442 (1998); Tetrahedron Lett., Bd. 35, S. 8761 (1994) .

Die Umsetzung von IB mit Alkali- oder Erdalkaliisocyanaten, insbesondere Natriumisocyanat, zu Harnstoffderivaten I.2 erfolgt unter den aus J. Med. Chem., Bd. 32, S. 990-997 (1989) bekannten Bedingungen.

Die Umsetzung von IB mit Methylisocyanat zu Harnstoffderivaten I.3 erfolgt unter den aus J. Org. Chem., Bd. 26, S. 5238 (1961) bekannten Bedingungen.

Die Umsetzung von IB mit Dimethylamin und Phosgen oder einem Phosgenäquivalent, wie Di- oder Triphosgen zu Harnstoffderivaten I.4 erfolgt unter den aus J. Chem. Res. Synop, 442 (1998) bekannten Bedingungen. Aus praktischen Gründen ist die Verwendung von Di- oder Triphosgen bevorzugt.

Die Umsetzung von IB zu Harnstoffderivaten I.5 mit Kohlenmonoxid und Methanol erfolgt unter Übergangsmetallkatalyse unter den aus Tetrahedron Lett., Bd. 35, S. 8761 (1994) bekannten Bedingungen.

Verbindungen der Formel I, in denen R¹ für NH-CO-A, worin A für C₁-C₄-Alkyl steht, können aus den Aminophenolverbindungen der Formel IB durch Acylierung mit Alkylcarbonsäurederivaten der Formel IV, in der R⁴ für C₁-C₄-Alkyl und Z für eine nucleophile Abgangsgruppe, wie Alkoxy oder Halogen steht, erhalten werden. Die Acylaminoderivate werden durch die Formel I.6 beschrieben, in der R⁴ für C₁-C₄-Alkyl steht

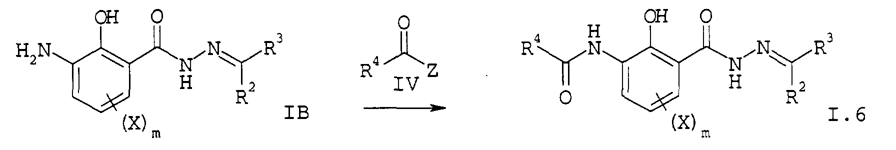

Die Acylierung von V erfolgt unter an sich literaturbekannten Bedingungen, sie erfolgt üblicherweise bei Temperaturen von -20°C bis +80°C, vorzugsweise 0°C bis +60°C, in einem inerten organischen Lösungsmittel in Gegenwart einer Base [vgl. Organikum, 15. Aufl., S. 508 ff., VEB Deutscher Verlag der Wissenschaften Berlin (1981)].

Geeignete Lösungsmittel sind Wasser, aliphatische Kohlenwasserstoffe, aromatische Kohlenwasserstoffe, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile, Ketone, Alkohole, sowie Dimethylsulfoxid, Dimethylformamid und Dimethylacetamid, besonders bevorzugt Tetrahydrofuran und Methylenchlorid. Es können auch Gemische der genannten Lösungsmittel verwendet werden.

Als Basen kommen allgemein anorganische Verbindungen, wie Alkalimetall- und Erdalkalimetallhydroxide, Alkalimetall- und Erdalkalimetalloxide, Alkalimetall- und Erdalkalimetallhydride, Alkalimetall- und Erdalkalimetallcarbonate, sowie Alkalimetallhydrogencarbonate, sowie Alkalimetall- und Erdalkalimetallalkoholate, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Pyridin und Triethylamin. Die Basen werden im allgemeinen äquimolar, im Überschuß oder gegebenenfalls als Lösungsmittel verwendet.

Die Edukte werden im allgemeinen in äquimolaren Mengen miteinander umgesetzt. Es kann für die Ausbeute vorteilhaft sein, den oder die Reaktanden in einem Überschuß bezogen auf IB einzusetzen.

Sofern einzelne Verbindungen I nicht auf den voranstehend beschriebenen Wegen zugänglich sind, können sie durch Derivatisierung anderer Verbindungen I hergestellt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 6 Kohlenstoffatomen (wie vorstehend genannt), welche über ein Sauerstoffatom (-O-) an das Gerüst gebunden sind;
**Alkenyl:** ungesättigte, geradkettige oder verzweigte Kohlenwasserstoffreste mit 2 bis 4 oder 6 Kohlenstoffatomen und einer Doppelbindung in einer beliebigen Position, wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Alkinyl:** geradkettige oder verzweigte Kohlenwasserstoffgruppen mit 2 bis 4 oder 6 Kohlenstoffatomen und einer Dreifachbindung in einer beliebigen Position, wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalkyl:** monocyclische, gesättigte Kohlenwasserstoffgruppen mit 3 bis 6 Kohlenstoffringgliedern wie Cyclopropyl, Cyclobutyl, Cyclopentyl und Cyclohexyl;
**5- oder 6-gliedriges Heterocyclyl** enthält neben Kohlenstoffringgliedern ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
**5-gliedriges Heteroaryl** enthält ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**6-gliedriges Heteroaryl** enthält ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
**Alkylen:** divalente unverzweigte Ketten aus 1 bis 4 CH₂-Gruppen, z.B. CH₂, CH₂CH₂, CH₂CH₂CH₂ und CH₂CH₂CH₂CH₂;
**Alkylendioxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O.

Im Hinblick auf ihre bestimmungsgemäße Verwendung der Salicylsäurehydrazid-Derivate der Formel I sind die folgenden Bedeutungen der Substituenten, und zwar jeweils für sich allein oder in Kombination, besonders bevorzugt:

Verbindungen der Formel IA sind besonders bevorzugt.

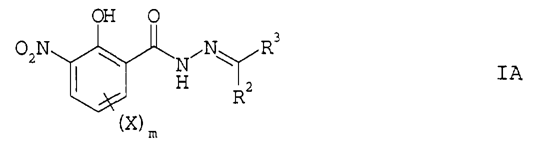

Verbindungen der Formel I, in der R¹ NH-CO-A bedeutet, sind besonders bevorzugt; diese Verbindungen werden durch die Formel IC beschrieben:

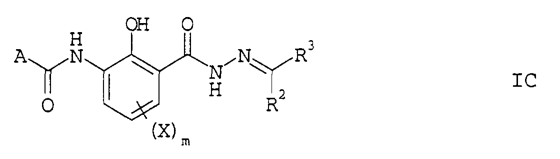

Insbesondere werden auch Verbindungen I.1 bevorzugt.

Gleichermaßen werden auch Verbindungen der Formel I.6 bevorzugt, in der R⁴ für Methyl steht; diese Verbindungen werden durch die Formel I.6a beschrieben:

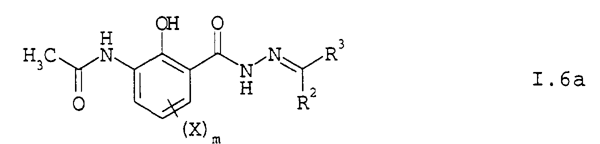

Daneben werden Verbindungen I besonders bevorzugt, in denen der Index m Null bedeutet; Diese Verbindungen werden durch die Formel ID beschrieben:

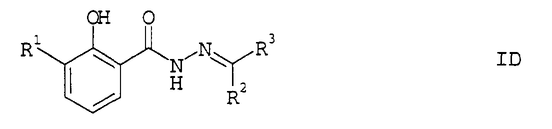

Außerdem werden Verbindungen I besonders bevorzugt, in denen (X)ₘ para-ständig zu der Phenol-OH-Gruppe steht und Halogen, NO₂, CN und C₁-C₄-Alkoxy, insbesondere 4-Chlor, 4-Brom und 4-NO₂, bedeutet.

Gleichermaßen besonders bevorzugt sind Verbindungen I, in denen R² für Wasserstoff, Methyl, Ethyl, n-Propyl, iso-Propyl und Cyano, insbesondere für Wasserstoff und Methyl steht.

Insbesondere werden auch Verbindungen I bevorzugt, in denen R³ für gegebenenfalls durch einen bis drei Reste R^{b} substituiertes Cyclohexyl und Phenyl, insbesondere Phenyl steht. Bei Verbindungen, in denen R³ Cyclohexyl bedeutet, kann der Substituent R^{b} E- oder Z-ständig in Bezug zu der C-R³-Bindung stehen.

Außerdem werden Verbindungen I besonders bevorzugt, in denen R³ für gegebenenfalls durch einen bis drei Reste R^{b} substituiertes Pyrazolyl, Pyrimidinyl, Pyridinyl, Pyrazinyl und Thiophenyl steht.

Die besonders bevorzugten Ausführungsformen der Zwischenprodukte in Bezug auf die Variablen entsprechen denen der Reste (X)ₘ, R¹, A, R² und R³ der Formel I.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel IA, in denen m Null bedeutet, R² für Wasserstoff steht und der Rest R³ für eine Verbindung jeweils einer Zeile der Tabelle A entspricht

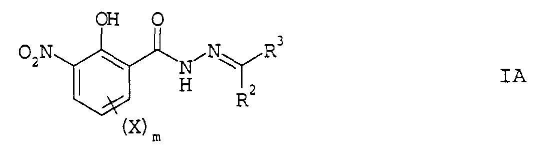

### Tabelle 2

Verbindungen der Formel IA, in denen m Null bedeutet, R² für Methyl steht und der Rest R³ für eine Verbindung jeweils einer Zeile der Tabelle A entspricht

### Tabelle 3

Verbindungen der Formel IA, in denen m Null bedeutet, R² für Ethyl steht und der Rest R³ für eine Verbindung jeweils einer Zeile der Tabelle A entspricht

### Tabelle 4

Verbindungen der Formel IA, in denen m Null bedeutet, R² für n-Propyl steht und der Rest R³ für eine Verbindung jeweils einer Zeile der Tabelle A entspricht

### Tabelle 5

Verbindungen der Formel IA, in denen m Null bedeutet, R² für iso-Propyl steht und der Rest R³ für eine Verbindung jeweils einer Zeile der Tabelle A entspricht

### Tabelle 6

Verbindungen der Formel IA, in denen m Null bedeutet, R² für Cyano steht und der Rest R³ für eine Verbindung jeweils einer Zeile der Tabelle A entspricht

### Tabelle 7

Verbindungen der Formel IA, in denen (X)ₘ 3-Chlor bedeutet, R² für Wasserstoff steht und der Rest R³ für eine Verbindung jeweils einer Zeile der Tabelle A entspricht

### Tabelle 8

Verbindungen der Formel IA, in denen (X)ₘ 3-Chlor bedeutet, R² für Methyl steht und der Rest R³ für eine Verbindung jeweils einer Zeile der Tabelle A entspricht

### Tabelle 9

Verbindungen der Formel IA, in denen (X)ₘ 3-Chlor bedeutet, R² für Ethyl steht und der Rest R³ für eine Verbindung jeweils einer Zeile der Tabelle A entspricht

### Tabelle 10

Verbindungen der Formel IA, in denen (X)ₘ 3-Chlor bedeutet, R² für n-Propyl steht und der Rest R³ für eine Verbindung jeweils einer Zeile der Tabelle A entspricht

### Tabelle 11

Verbindungen der Formel IA, in denen (X)ₘ 3-Chlor bedeutet, R² für iso-Propyl steht und der Rest R³ für eine Verbindung jeweils einer Zeile der Tabelle A entspricht

### Tabelle 12

Verbindungen der Formel IA, in denen (X)ₘ 3-Chlor bedeutet, R² für Cyano steht und der Rest R³ für eine Verbindung jeweils einer Zeile der Tabelle A entspricht

### Tabelle 13

Verbindungen der Formel IA, in denen (X)ₘ 4-Chlor bedeutet, R² für Wasserstoff steht und der Rest R³ für eine Verbindung jeweils einer Zeile der Tabelle A entspricht

### Tabelle 14

Verbindungen der Formel IA, in denen (X)ₘ 4-Chlor bedeutet, R² für Methyl steht und der Rest R³ für eine Verbindung jeweils einer Zeile der Tabelle A entspricht

### Tabelle 15

Verbindungen der Formel IA, in denen (X)ₘ 4-Chlor bedeutet, R² für Ethyl steht und der Rest R³ für eine Verbindung jeweils einer Zeile der Tabelle A entspricht

### Tabelle 16

Verbindungen der Formel IA, in denen (X)ₘ 4-Chlor bedeutet, R² für n-Propyl steht und der Rest R³ für eine Verbindung jeweils einer Zeile der Tabelle A entspricht

### Tabelle 17

Verbindungen der Formel IA, in denen (X)ₘ 4-Chlor bedeutet, R² für iso-Propyl steht und der Rest R³ für eine Verbindung jeweils einer Zeile der Tabelle A entspricht

### Tabelle 18

Verbindungen der Formel IA, in denen (X)ₘ 4-Chlor bedeutet, R² für Cyano steht und der Rest R³ für eine Verbindung jeweils einer Zeile der Tabelle A entspricht

### Tabelle 19

Verbindungen der Formel IA, in denen (X)ₘ 5-Chlor bedeutet, R² für Wasserstoff steht und der Rest R³ für eine Verbindung jeweils einer Zeile der Tabelle A entspricht

### Tabelle 20

Verbindungen der Formel IA, in denen (X)ₘ 5-Chlor bedeutet, R² für Methyl steht und der Rest R³ für eine Verbindung jeweils einer Zeile der Tabelle A entspricht

### Tabelle 21

Verbindungen der Formel IA, in denen (X)ₘ 5-Chlor bedeutet, R² für Ethyl steht und der Rest R³ für eine Verbindung jeweils einer Zeile der Tabelle A entspricht

### Tabelle 22

Verbindungen der Formel IA, in denen (X)ₘ 5-Chlor bedeutet, R² für n-Propyl steht und der Rest R³ für eine Verbindung jeweils einer Zeile der Tabelle A entspricht

### Tabelle 23

Verbindungen der Formel IA, in denen (X)ₘ 5-Chlor bedeutet, R² für iso-Propyl steht und der Rest R³ für eine Verbindung jeweils einer Zeile der Tabelle A entspricht

### Tabelle 24

Verbindungen der Formel IA, in denen (X)ₘ 5-Chlor bedeutet, R² für Cyano steht und der Rest R³ für eine Verbindung jeweils einer Zeile der Tabelle A entspricht

### Tabelle 25

Verbindungen der Formel IA, in denen (X)ₘ 3-Brom bedeutet, R² für Wasserstoff steht und der Rest R³ für eine Verbindung jeweils einer Zeile der Tabelle A entspricht

### Tabelle 26

Verbindungen der Formel IA, in denen (X)ₘ 3-Brom bedeutet, R² für Methyl steht und der Rest R³ für eine Verbindung jeweils einer Zeile der Tabelle A entspricht

### Tabelle 27

Verbindungen der Formel IA, in denen (X)ₘ 3-Brom bedeutet, R² für Ethyl steht und der Rest R³ für eine Verbindung jeweils einer Zeile der Tabelle A entspricht

### Tabelle 28

Verbindungen der Formel IA, in denen (X)ₘ 3-Brom bedeutet, R² für n-Propyl steht und der Rest R³ für eine Verbindung jeweils einer Zeile der Tabelle A entspricht

### Tabelle 29

Verbindungen der Formel IA, in denen (X)ₘ 3-Brom bedeutet, R² für iso-Propyl steht und der Rest R³ für eine Verbindung jeweils einer Zeile der Tabelle A entspricht

### Tabelle 30

Verbindungen der Formel IA, in denen (X)ₘ 3-Brom bedeutet, R² für Cyano steht und der Rest R³ für eine Verbindung jeweils einer Zeile der Tabelle A entspricht

### Tabelle 31

Verbindungen der Formel IA, in denen (X)ₘ 4-Brom bedeutet, R² für Wasserstoff steht und der Rest R³ für eine Verbindung jeweils einer Zeile der Tabelle A entspricht

### Tabelle 32

Verbindungen der Formel IA, in denen (X)ₘ 4-Brom bedeutet, R² für Methyl steht und der Rest R³ für eine Verbindung jeweils einer Zeile der Tabelle A entspricht

### Tabelle 33

Verbindungen der Formel IA, in denen (X)ₘ 4-Brom bedeutet, R² für Ethyl steht und der Rest R³ für eine Verbindung jeweils einer Zeile der Tabelle A entspricht

### Tabelle 34

Verbindungen der Formel IA, in denen (X)ₘ 4-Brom bedeutet, R² für n-Propyl steht und der Rest R³ für eine Verbindung jeweils einer Zeile der Tabelle A entspricht

### Tabelle 35

Verbindungen der Formel IA, in denen (X)ₘ 4-Brom bedeutet, R² für iso-Propyl steht und der Rest R³ für eine Verbindung jeweils einer Zeile der Tabelle A entspricht

### Tabelle 36

Verbindungen der Formel IA, in denen (X)ₘ 4-Brom bedeutet, R² für Cyano steht und der Rest R³ für eine Verbindung jeweils einer Zeile der Tabelle A entspricht

### Tabelle 37

Verbindungen der Formel IA, in denen (X)ₘ 5-Brom bedeutet, R² für Wasserstoff steht und der Rest R³ für eine Verbindung jeweils einer Zeile der Tabelle A entspricht

### Tabelle 38

Verbindungen der Formel IA, in denen (X)ₘ 5-Brom bedeutet, R² für Methyl steht und der Rest R³ für eine Verbindung jeweils einer Zeile der Tabelle A entspricht

### Tabelle 39

Verbindungen der Formel IA, in denen (X)ₘ 5-Brom bedeutet, R² für Ethyl steht und der Rest R³ für eine Verbindung jeweils einer Zeile der Tabelle A entspricht

### Tabelle 40

Verbindungen der Formel IA, in denen (X)ₘ 5-Brom bedeutet, R² für n-Propyl steht und der Rest R³ für eine Verbindung jeweils einer Zeile der Tabelle A entspricht

### Tabelle 41

Verbindungen der Formel IA, in denen (X)ₘ 5-Brom bedeutet, R² für iso-Propyl steht und der Rest R³ für eine Verbindung jeweils einer Zeile der Tabelle A entspricht

### Tabelle 42

Verbindungen der Formel IA, in denen (X)ₘ 5-Brom bedeutet, R² für Cyano steht und der Rest R³ für eine Verbindung jeweils einer Zeile der Tabelle A entspricht

### Tabelle 43

Verbindungen der Formel IA, in denen (X)ₘ 3-Nitro bedeutet, R² für Wasserstoff steht und der Rest R³ für eine Verbindung jeweils einer Zeile der Tabelle A entspricht

### Tabelle 44

Verbindungen der Formel IA, in denen (X)ₘ 3-Nitro bedeutet, R² für Methyl steht und der Rest R³ für eine Verbindung jeweils einer Zeile der Tabelle A entspricht

### Tabelle 45

Verbindungen der Formel IA, in denen (X)ₘ 3-Nitro bedeutet, R² für Ethyl steht und der Rest R³ für eine Verbindung jeweils einer Zeile der Tabelle A entspricht

### Tabelle 46

Verbindungen der Formel IA, in denen (X)ₘ 3-Nitro bedeutet, R² für n-Propyl steht und der Rest R³ für eine Verbindung jeweils einer Zeile der Tabelle A entspricht

### Tabelle 47

Verbindungen der Formel IA, in denen (X)ₘ 3-Nitro bedeutet, R² für iso-Propyl steht und der Rest R³ für eine Verbindung jeweils einer Zeile der Tabelle A entspricht

### Tabelle 48

Verbindungen der Formel IA, in denen (X)ₘ 3-Nitro bedeutet, R² für Cyano steht und der Rest R³ für eine Verbindung jeweils einer Zeile der Tabelle A entspricht

### Tabelle 49

Verbindungen der Formel IA, in denen (X)ₘ 4-Nitro bedeutet, R² für Wasserstoff steht und der Rest R³ für eine Verbindung jeweils einer Zeile der Tabelle A entspricht

### Tabelle 50

Verbindungen der Formel IA, in denen (X)ₘ 4-Nitro bedeutet, R² für Methyl steht und der Rest R³ für eine Verbindung jeweils einer Zeile der Tabelle A entspricht

### Tabelle 51

Verbindungen der Formel IA, in denen (X)ₘ 4-Nitro bedeutet, R² für Ethyl steht und der Rest R³ für eine Verbindung jeweils einer Zeile der Tabelle A entspricht

### Tabelle 52

Verbindungen der Formel IA, in denen (X)ₘ 4-Nitro bedeutet, R² für n-Propyl steht und der Rest R³ für eine Verbindung jeweils einer Zeile der Tabelle A entspricht

### Tabelle 53

Verbindungen der Formel IA, in denen (X)ₘ 4-Nitro bedeutet, R² für iso-Propyl steht und der Rest R³ für eine Verbindung jeweils einer Zeile der Tabelle A entspricht

### Tabelle 54

Verbindungen der Formel IA, in denen (X)ₘ 4-Nitro bedeutet, R² für Cyano steht und der Rest R³ für eine Verbindung jeweils einer Zeile der Tabelle A entspricht

### Tabelle 55

Verbindungen der Formel IA, in denen (X)ₘ 5-Nitro bedeutet, R² für Wasserstoff steht und der Rest R³ für eine Verbindung jeweils einer Zeile der Tabelle A entspricht

### Tabelle 56

Verbindungen der Formel IA, in denen (X)ₘ 5-Nitro bedeutet, R² für Methyl steht und der Rest R³ für eine Verbindung jeweils einer Zeile der Tabelle A entspricht

### Tabelle 57

Verbindungen der Formel IA, in denen (X)ₘ 5-Nitro bedeutet, R² für Ethyl steht und der Rest R³ für eine Verbindung jeweils einer Zeile der Tabelle A entspricht

### Tabelle 58

Verbindungen der Formel IA, in denen (X)ₘ 5-Nitro bedeutet, R² für n-Propyl steht und der Rest R³ für eine Verbindung jeweils einer Zeile der Tabelle A entspricht

### Tabelle 59

Verbindungen der Formel IA, in denen (X)ₘ 5-Nitro bedeutet, R² für iso-Propyl steht und der Rest R³ für eine Verbindung jeweils einer Zeile der Tabelle A entspricht

### Tabelle 60

Verbindungen der Formel IA, in denen (X)ₘ 5-Nitro bedeutet, R² für Cyano steht und der Rest R³ für eine Verbindung jeweils einer Zeile der Tabelle A entspricht

### Tabelle 61

Verbindungen der Formel IB, in denen m Null bedeutet, R² für Wasserstoff steht und der Rest R³ für eine Verbindung jeweils einer Zeile der Tabelle A entspricht

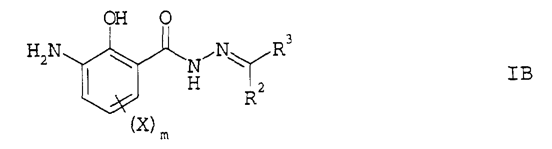

### Tabelle 62

Verbindungen der Formel IB, in denen m Null bedeutet, R² für Methyl steht und der Rest R³ für eine Verbindung jeweils einer Zeile der Tabelle A entspricht

### Tabelle 63

Verbindungen der Formel IB, in denen m Null bedeutet, R² für Ethyl steht und der Rest R³ für eine Verbindung jeweils einer Zeile der Tabelle A entspricht

### Tabelle 64

Verbindungen der Formel IB, in denen m Null bedeutet, R² für n-Propyl steht und der Rest R³ für eine Verbindung jeweils einer Zeile der Tabelle A entspricht

### Tabelle 65

Verbindungen der Formel IB, in denen m Null bedeutet, R² für iso-Propyl steht und der Rest R³ für eine Verbindung jeweils einer Zeile der Tabelle A entspricht

### Tabelle 66

Verbindungen der Formel IB, in denen m Null bedeutet, R² für Cyano steht und der Rest R³ für eine Verbindung jeweils einer Zeile der Tabelle A entspricht

### Tabelle 67

Verbindungen der Formel IB, in denen (X)ₘ 3-Chlor bedeutet, R² für Wasserstoff steht und der Rest R³ für eine Verbindung jeweils einer Zeile der Tabelle A entspricht

### Tabelle 68

Verbindungen der Formel IB, in denen (X)ₘ 3-Chlor bedeutet, R² für Methyl steht und der Rest R³ für eine Verbindung jeweils einer Zeile der Tabelle A entspricht

### Tabelle 69

Verbindungen der Formel IB, in denen (X)ₘ 3-Chlor bedeutet, R² für Ethyl steht und der Rest R³ für eine Verbindung jeweils einer Zeile der Tabelle A entspricht

### Tabelle 70

Verbindungen der Formel IB, in denen (X)ₘ 3-Chlor bedeutet, R² für n-Propyl steht und der Rest R³ für eine Verbindung jeweils einer Zeile der Tabelle A entspricht

### Tabelle 71

Verbindungen der Formel IB, in denen (X)ₘ 3-Chlor bedeutet, R² für iso-Propyl steht und der Rest R³ für eine Verbindung jeweils einer Zeile der Tabelle A entspricht

### Tabelle 72

Verbindungen der Formel IB, in denen (X)ₘ 3-Chlor bedeutet, R² für Cyano steht und der Rest R³ für eine Verbindung jeweils einer Zeile der Tabelle A entspricht

### Tabelle 73

Verbindungen der Formel IB, in denen (X)ₘ 4-Chlor bedeutet, R² für Wasserstoff steht und der Rest R³ für eine Verbindung jeweils einer Zeile der Tabelle A entspricht

### Tabelle 74

Verbindungen der Formel IB, in denen (X)ₘ 4-Chlor bedeutet, R² für Methyl steht und der Rest R³ für eine Verbindung jeweils einer Zeile der Tabelle A entspricht

### Tabelle 75

Verbindungen der Formel IB, in denen (X)ₘ 4-Chlor bedeutet, R² für Ethyl steht und der Rest R³ für eine Verbindung jeweils einer Zeile der Tabelle A entspricht

### Tabelle 76

Verbindungen der Formel IB, in denen (X)ₘ 4-Chlor bedeutet, R² für n-Propyl steht und der Rest R³ für eine Verbindung jeweils einer Zeile der Tabelle A entspricht

### Tabelle 77

Verbindungen der Formel IB, in denen (X)ₘ 4-Chlor bedeutet, R² für iso-Propyl steht und der Rest R³ für eine Verbindung jeweils einer Zeile der Tabelle A entspricht

### Tabelle 78

Verbindungen der Formel IB, in denen (X)ₘ 4-Chlor bedeutet, R² für Cyano steht und der Rest R³ für eine Verbindung jeweils einer Zeile der Tabelle A entspricht

### Tabelle 79

Verbindungen der Formel IB, in denen (X)ₘ 5-Chlor bedeutet, R² für Wasserstoff steht und der Rest R³ für eine Verbindung jeweils einer Zeile der Tabelle A entspricht

### Tabelle 80

Verbindungen der Formel IB, in denen (X)ₘ 5-Chlor bedeutet, R² für Methyl steht und der Rest R³ für eine Verbindung jeweils einer Zeile der Tabelle A entspricht

### Tabelle 81

Verbindungen der Formel IB, in denen (X)ₘ 5-Chlor bedeutet, R² für Ethyl steht und der Rest R³ für eine Verbindung jeweils einer Zeile der Tabelle A entspricht

### Tabelle 82

Verbindungen der Formel IB, in denen (X)ₘ 5-Chlor bedeutet, R² für n-Propyl steht und der Rest R³ für eine Verbindung jeweils einer Zeile der Tabelle A entspricht

### Tabelle 83

Verbindungen der Formel IB, in denen (X)ₘ 5-Chlor bedeutet, R² für iso-Propyl steht und der Rest R³ für eine Verbindung jeweils einer Zeile der Tabelle A entspricht

### Tabelle 84

Verbindungen der Formel IB, in denen (X)ₘ 5-Chlor bedeutet, R² für Cyano steht und der Rest R³ für eine Verbindung jeweils einer Zeile der Tabelle A entspricht

### Tabelle 85

Verbindungen der Formel IB, in denen (X)ₘ 3-Brom bedeutet, R² für Wasserstoff steht und der Rest R³ für eine Verbindung jeweils einer Zeile der Tabelle A entspricht

### Tabelle 86

Verbindungen der Formel IB, in denen (X)ₘ 3-Brom bedeutet, R² für Methyl steht und der Rest R³ für eine Verbindung jeweils einer Zeile der Tabelle A entspricht

### Tabelle 87

Verbindungen der Formel IB, in denen (X)ₘ 3-Brom bedeutet, R² für Ethyl steht und der Rest R³ für eine Verbindung jeweils einer Zeile der Tabelle A entspricht

### Tabelle 88

Verbindungen der Formel IB, in denen (X)ₘ 3-Brom bedeutet, R² für n-Propyl steht und der Rest R³ für eine Verbindung jeweils einer Zeile der Tabelle A entspricht

### Tabelle 89

Verbindungen der Formel IB, in denen (X)ₘ 3-Brom bedeutet, R² für iso-Propyl steht und der Rest R³ für eine Verbindung jeweils einer Zeile der Tabelle A entspricht

### Tabelle 90

Verbindungen der Formel IB, in denen (X)ₘ 3-Brom bedeutet, R² für Cyano steht und der Rest R³ für eine Verbindung jeweils einer Zeile der Tabelle A entspricht

### Tabelle 91

Verbindungen der Formel IB, in denen (X)ₘ 4-Brom bedeutet, R² für Wasserstoff steht und der Rest R³ für eine Verbindung jeweils einer Zeile der Tabelle A entspricht

### Tabelle 92

Verbindungen der Formel IB, in denen (X)ₘ 4-Brom bedeutet, R² für Methyl steht und der Rest R³ für eine Verbindung jeweils einer Zeile der Tabelle A entspricht

### Tabelle 93

Verbindungen der Formel IB, in denen (X)ₘ 4-Brom bedeutet, R² für Ethyl steht und der Rest R³ für eine Verbindung jeweils einer Zeile der Tabelle A entspricht

### Tabelle 94

Verbindungen der Formel IB, in denen (X)ₘ 4-Brom bedeutet, R² für n-Propyl steht und der Rest R³ für eine Verbindung jeweils einer Zeile der Tabelle A entspricht

### Tabelle 95

Verbindungen der Formel IB, in denen (X)ₘ 4-Brom bedeutet, R² für iso-Propyl steht und der Rest R³ für eine Verbindung jeweils einer Zeile der Tabelle A entspricht

### Tabelle 96

Verbindungen der Formel IB, in denen (X)ₘ 4-Brom bedeutet, R² für Cyano steht und der Rest R³ für eine Verbindung jeweils einer Zeile der Tabelle A entspricht

### Tabelle 97

Verbindungen der Formel IB, in denen (X)ₘ 5-Brom bedeutet, R² für Wasserstoff steht und der Rest R³ für eine Verbindung jeweils einer Zeile der Tabelle A entspricht

### Tabelle 98

Verbindungen der Formel IB, in denen (X)ₘ 5-Brom bedeutet, R² für Methyl steht und der Rest R³ für eine Verbindung jeweils einer Zeile der Tabelle A entspricht

### Tabelle 99

Verbindungen der Formel IB, in denen (X)ₘ 5-Brom bedeutet, R² für Ethyl steht und der Rest R³ für eine Verbindung jeweils einer Zeile der Tabelle A entspricht

### Tabelle 100

Verbindungen der Formel IB, in denen (X)ₘ 5-Brom bedeutet, R² für n-Propyl steht und der Rest R³ für eine Verbindung jeweils einer Zeile der Tabelle A entspricht

### Tabelle 101

Verbindungen der Formel IB, in denen (X)ₘ 5-Brom bedeutet, R² für iso-Propyl steht und der Rest R³ für eine Verbindung jeweils einer Zeile der Tabelle A entspricht

### Tabelle 102

Verbindungen der Formel IB, in denen (X)ₘ 5-Brom bedeutet, R² für Cyano steht und der Rest R³ für eine Verbindung jeweils einer Zeile der Tabelle A entspricht

### Tabelle 103

Verbindungen der Formel IB, in denen (X)ₘ 3-Nitro bedeutet, R² für Wasserstoff steht und der Rest R³ für eine Verbindung jeweils einer Zeile der Tabelle A entspricht

### Tabelle 104

Verbindungen der Formel IB, in denen (X)ₘ 3-Nitro bedeutet, R² für Methyl steht und der Rest R³ für eine Verbindung jeweils einer Zeile der Tabelle A entspricht

### Tabelle 105

Verbindungen der Formel IB, in denen (X)ₘ 3-Nitro bedeutet, R² für Ethyl steht und der Rest R³ für eine Verbindung jeweils einer Zeile der Tabelle A entspricht

### Tabelle 106

Verbindungen der Formel IB, in denen (X)ₘ 3-Nitro bedeutet, R² für n-Propyl steht und der Rest R³ für eine Verbindung jeweils einer Zeile der Tabelle A entspricht

### Tabelle 107

Verbindungen der Formel IB, in denen (X)ₘ 3-Nitro bedeutet, R² für iso-Propyl steht und der Rest R³ für eine Verbindung jeweils einer Zeile der Tabelle A entspricht

### Tabelle 108

Verbindungen der Formel IB, in denen (X)ₘ 3-Nitro bedeutet, R² für Cyano steht und der Rest R³ für eine Verbindung jeweils einer Zeile der Tabelle A entspricht

### Tabelle 109

Verbindungen der Formel IB, in denen (X)ₘ 4-Nitro bedeutet, R² für Wasserstoff steht und der Rest R³ für eine Verbindung jeweils einer Zeile der Tabelle A entspricht

### Tabelle 110

Verbindungen der Formel IB, in denen (X)ₘ 4-Nitro bedeutet, R² für Methyl steht und der Rest R³ für eine Verbindung jeweils einer Zeile der Tabelle A entspricht

### Tabelle 111

Verbindungen der Formel IB, in denen (X)ₘ 4-Nitro bedeutet, R² für Ethyl steht und der Rest R³ für eine Verbindung jeweils einer Zeile der Tabelle A entspricht

### Tabelle 112

Verbindungen der Formel IB, in denen (X)ₘ 4-Nitro bedeutet, R² für n-Propyl steht und der Rest R³ für eine Verbindung jeweils einer Zeile der Tabelle A entspricht

### Tabelle 113

Verbindungen der Formel IB, in denen (X)ₘ 4-Nitro bedeutet, R² für iso-Propyl steht und der Rest R³ für eine Verbindung jeweils einer Zeile der Tabelle A entspricht

### Tabelle 114

Verbindungen der Formel IB, in denen (X)ₘ 4-Nitro bedeutet, R² für Cyano steht und der Rest R³ für eine Verbindung jeweils einer Zeile der Tabelle A entspricht

### Tabelle 115

Verbindungen der Formel IB, in denen (X)ₘ 5-Nitro bedeutet, R² für Wasserstoff steht und der Rest R³ für eine Verbindung jeweils einer Zeile der Tabelle A entspricht

### Tabelle 116

Verbindungen der Formel IB, in denen (X)ₘ 5-Nitro bedeutet, R² für Methyl steht und der Rest R³ für eine Verbindung jeweils einer Zeile der Tabelle A entspricht

### Tabelle 117

Verbindungen der Formel IB, in denen (X)ₘ 5-Nitro bedeutet, R² für Ethyl steht und der Rest R³ für eine Verbindung jeweils einer Zeile der Tabelle A entspricht

### Tabelle 118

Verbindungen der Formel IB, in denen (X)ₘ 5-Nitro bedeutet, R² für n-Propyl steht und der Rest R³ für eine Verbindung jeweils einer Zeile der Tabelle A entspricht

### Tabelle 119

Verbindungen der Formel IB, in denen (X)ₘ 5-Nitro bedeutet, R² für iso-Propyl steht und der Rest R³ für eine Verbindung jeweils einer Zeile der Tabelle A entspricht

### Tabelle 120

Verbindungen der Formel IB, in denen (X)ₘ 5-Nitro bedeutet, R² für Cyano steht und der Rest R³ für eine Verbindung jeweils einer Zeile der Tabelle A entspricht

### Tabelle 121

Verbindungen der Formel I.1, in denen m Null bedeutet, R² für Wasserstoff steht und der Rest R³ für eine Verbindung jeweils einer Zeile der Tabelle A entspricht

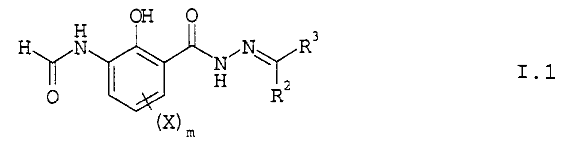

### Tabelle 122

Verbindungen der Formel I.1, in denen m Null bedeutet, R² für Methyl steht und der Rest R³ für eine Verbindung jeweils einer Zeile der Tabelle A entspricht

### Tabelle 123

Verbindungen der Formel I.1, in denen m Null bedeutet, R² für Ethyl steht und der Rest R³ für eine Verbindung jeweils einer Zeile der Tabelle A entspricht

### Tabelle 124

Verbindungen der Formel I.1, in denen m Null bedeutet, R² für n-Propyl steht und der Rest R³ für eine Verbindung jeweils einer Zeile der Tabelle A entspricht

### Tabelle 125

Verbindungen der Formel I.1, in denen m Null bedeutet, R² für iso-Propyl steht und der Rest R³ für eine Verbindung jeweils einer Zeile der Tabelle A entspricht

### Tabelle 126

Verbindungen der Formel I.1, in denen m Null bedeutet, R² für Cyano steht und der Rest R³ für eine Verbindung jeweils einer Zeile der Tabelle A entspricht

### Tabelle 127

Verbindungen der Formel I.1, in denen (X)ₘ 3-Chlor bedeutet, R² für Wasserstoff steht und der Rest R³ für eine Verbindung jeweils einer Zeile der Tabelle A entspricht

### Tabelle 128

Verbindungen der Formel I.1, in denen (X)ₘ 3-Chlor bedeutet, R² für Methyl steht und der Rest R³ für eine Verbindung jeweils einer Zeile der Tabelle A entspricht

### Tabelle 129

Verbindungen der Formel I.1, in denen (X)ₘ 3-Chlor bedeutet, R² für Ethyl steht und der Rest R³ für eine Verbindung jeweils einer Zeile der Tabelle A entspricht

### Tabelle 130

Verbindungen der Formel I.1, in denen (X)ₘ 3-Chlor bedeutet, R² für n-Propyl steht und der Rest R³ für eine Verbindung jeweils einer Zeile der Tabelle A entspricht

### Tabelle 131

Verbindungen der Formel I.1, in denen (X)ₘ 3-Chlor bedeutet, R² für iso-Propyl steht und der Rest R³ für eine Verbindung jeweils einer Zeile der Tabelle A entspricht

### Tabelle 132

Verbindungen der Formel I.1, in denen (X)ₘ 3-Chlor bedeutet, R² für Cyano steht und der Rest R³ für eine Verbindung jeweils einer Zeile der Tabelle A entspricht

### Tabelle 133

Verbindungen der Formel I.1, in denen (X)ₘ 4-Chlor bedeutet, R² für Wasserstoff steht und der Rest R³ für eine Verbindung jeweils einer Zeile der Tabelle A entspricht

### Tabelle 134

Verbindungen der Formel I.1, in denen (X)ₘ 4-Chlor bedeutet, R² für Methyl steht und der Rest R³ für eine Verbindung jeweils einer Zeile der Tabelle A entspricht

### Tabelle 135

Verbindungen der Formel I.1, in denen (X)ₘ 4-Chlor bedeutet, R² für Ethyl steht und der Rest R³ für eine Verbindung jeweils einer Zeile der Tabelle A entspricht

### Tabelle 136

Verbindungen der Formel I.1, in denen (X)ₘ 4-Chlor bedeutet, R² für n-Propyl steht und der Rest R³ für eine Verbindung jeweils einer Zeile der Tabelle A entspricht

### Tabelle 137

Verbindungen der Formel I.1, in denen (X)ₘ 4-Chlor bedeutet, R² für iso-Propyl steht und der Rest R³ für eine Verbindung jeweils einer Zeile der Tabelle A entspricht

### Tabelle 138

Verbindungen der Formel I.1, in denen (X)ₘ 4-Chlor bedeutet, R² für Cyano steht und der Rest R³ für eine Verbindung jeweils einer Zeile der Tabelle A entspricht

### Tabelle 139

Verbindungen der Formel I.1, in denen (X)ₘ 5-Chlor bedeutet, R² für Wasserstoff steht und der Rest R³ für eine Verbindung jeweils einer Zeile der Tabelle A entspricht

### Tabelle 140

Verbindungen der Formel I.1, in denen (X)ₘ 5-Chlor bedeutet, R² für Methyl steht und der Rest R³ für eine Verbindung jeweils einer Zeile der Tabelle A entspricht

### Tabelle 141

Verbindungen der Formel I.1, in denen (X)ₘ 5-Chlor bedeutet, R² für Ethyl steht und der Rest R³ für eine Verbindung jeweils einer Zeile der Tabelle A entspricht

### Tabelle 142

Verbindungen der Formel I.1, in denen (X)ₘ 5-Chlor bedeutet, R² für n-Propyl steht und der Rest R³ für eine Verbindung jeweils einer Zeile der Tabelle A entspricht

### Tabelle 143

Verbindungen der Formel I.1, in denen (X)ₘ 5-Chlor bedeutet, R² für iso-Propyl steht und der Rest R³ für eine Verbindung jeweils einer Zeile der Tabelle A entspricht

### Tabelle 144

Verbindungen der Formel I.1, in denen (X)ₘ 5-Chlor bedeutet, R² für Cyano steht und der Rest R³ für eine Verbindung jeweils einer Zeile der Tabelle A entspricht

### Tabelle 145

Verbindungen der Formel I.1, in denen (X)ₘ 3-Brom bedeutet, R² für Wasserstoff steht und der Rest R³ für eine Verbindung jeweils einer Zeile der Tabelle A entspricht

### Tabelle 146

Verbindungen der Formel I.1, in denen (X)ₘ 3-Brom bedeutet, R² für Methyl steht und der Rest R³ für eine Verbindung jeweils einer Zeile der Tabelle A entspricht

### Tabelle 147

Verbindungen der Formel I.1, in denen (X)ₘ 3-Brom bedeutet, R² für Ethyl steht und der Rest R³ für eine Verbindung jeweils einer Zeile der Tabelle A entspricht

### Tabelle 148

Verbindungen der Formel I.1, in denen (X)ₘ 3-Brom bedeutet, R² für n-Propyl steht und der Rest R³ für eine Verbindung jeweils einer Zeile der Tabelle A entspricht

### Tabelle 149

Verbindungen der Formel I.1, in denen (X)ₘ 3-Brom bedeutet, R² für iso-Propyl steht und der Rest R³ für eine Verbindung jeweils einer Zeile der Tabelle A entspricht

### Tabelle 150

Verbindungen der Formel I.1, in denen (X)ₘ 3-Brom bedeutet, R² für Cyano steht und der Rest R³ für eine Verbindung jeweils einer Zeile der Tabelle A entspricht

### Tabelle 151

Verbindungen der Formel I.1, in denen (X)ₘ 4-Brom bedeutet, R² für Wasserstoff steht und der Rest R³ für eine Verbindung jeweils einer Zeile der Tabelle A entspricht

### Tabelle 152

Verbindungen der Formel I.1, in denen (X)ₘ 4-Brom bedeutet, R² für Methyl steht und der Rest R³ für eine Verbindung jeweils einer Zeile der Tabelle A entspricht

### Tabelle 153

Verbindungen der Formel I.1, in denen (X)ₘ 4-Brom bedeutet, R² für Ethyl steht und der Rest R³ für eine Verbindung jeweils einer Zeile der Tabelle A entspricht

### Tabelle 154

Verbindungen der Formel I.1, in denen (X)ₘ 4-Brom bedeutet, R² für n-Propyl steht und der Rest R³ für eine Verbindung jeweils einer Zeile der Tabelle A entspricht

### Tabelle 155

Verbindungen der Formel I.1, in denen (X)ₘ 4-Brom bedeutet, R² für iso-Propyl steht und der Rest R³ für eine Verbindung jeweils einer Zeile der Tabelle A entspricht

### Tabelle 156

Verbindungen der Formel I.1, in denen (X)ₘ 4-Brom bedeutet, R² für Cyano steht und der Rest R³ für eine Verbindung jeweils einer Zeile der Tabelle A entspricht

### Tabelle 157

Verbindungen der Formel I.1, in denen (X)ₘ 5-Brom bedeutet, R² für Wasserstoff steht und der Rest R³ für eine Verbindung jeweils einer Zeile der Tabelle A entspricht

### Tabelle 158

Verbindungen der Formel I.1, in denen (X)ₘ 5-Brom bedeutet, R² für Methyl steht und der Rest R³ für eine Verbindung jeweils einer Zeile der Tabelle A entspricht

### Tabelle 159

Verbindungen der Formel I.1, in denen (X)ₘ 5-Brom bedeutet, R² für Ethyl steht und der Rest R³ für eine Verbindung jeweils einer Zeile der Tabelle A entspricht

### Tabelle 160

Verbindungen der Formel I.1, in denen (X)ₘ 5-Brom bedeutet, R² für n-Propyl steht und der Rest R³ für eine Verbindung jeweils einer Zeile der Tabelle A entspricht

### Tabelle 161

Verbindungen der Formel I.1, in denen (X)ₘ 5-Brom bedeutet, R² für iso-Propyl steht und der Rest R³ für eine Verbindung jeweils einer Zeile der Tabelle A entspricht

### Tabelle 162

Verbindungen der Formel I.1, in denen (X)ₘ 5-Brom bedeutet, R² für Cyano steht und der Rest R³ für eine Verbindung jeweils einer Zeile der Tabelle A entspricht

### Tabelle 163

Verbindungen der Formel I.1, in denen (X)ₘ 3-Nitro bedeutet, R² für Wasserstoff steht und der Rest R³ für eine Verbindung jeweils einer Zeile der Tabelle A entspricht

### Tabelle 164

Verbindungen der Formel I.1, in denen (X)ₘ 3-Nitro bedeutet, R² für Methyl steht und der Rest R³ für eine Verbindung jeweils einer Zeile der Tabelle A entspricht

### Tabelle 165

Verbindungen der Formel I.1, in denen (X)ₘ 3-Nitro bedeutet, R² für Ethyl steht und der Rest R³ für eine Verbindung jeweils einer Zeile der Tabelle A entspricht

### Tabelle 166

Verbindungen der Formel I.1, in denen (X)ₘ 3-Nitro bedeutet, R² für n-Propyl steht und der Rest R³ für eine Verbindung jeweils einer Zeile der Tabelle A entspricht

### Tabelle 167

Verbindungen der Formel I.1, in denen (X)ₘ 3-Nitro bedeutet, R² für iso-Propyl steht und der Rest R³ für eine Verbindung jeweils einer Zeile der Tabelle A entspricht

### Tabelle 168

Verbindungen der Formel I.1, in denen (X)ₘ 3-Nitro bedeutet, R² für Cyano steht und der Rest R³ für eine Verbindung jeweils einer Zeile der Tabelle A entspricht

### Tabelle 169

Verbindungen der Formel I.1, in denen (X)ₘ 4-Nitro bedeutet, R² für Wasserstoff steht und der Rest R³ für eine Verbindung jeweils einer Zeile der Tabelle A entspricht

### Tabelle 170

Verbindungen der Formel I.1, in denen (X)ₘ 4-Nitro bedeutet, R² für Methyl steht und der Rest R³ für eine Verbindung jeweils einer Zeile der Tabelle A entspricht

### Tabelle 171

Verbindungen der Formel I.1, in denen (X)ₘ 4-Nitro bedeutet, R² für Ethyl steht und der Rest R³ für eine Verbindung jeweils einer Zeile der Tabelle A entspricht

### Tabelle 172

Verbindungen der Formel I.1, in denen (X)ₘ 4-Nitro bedeutet, R² für n-Propyl steht und der Rest R³ für eine Verbindung jeweils einer Zeile der Tabelle A entspricht

### Tabelle 173

Verbindungen der Formel I.1, in denen (X)ₘ 4-Nitro bedeutet, R² für iso-Propyl steht und der Rest R³ für eine Verbindung jeweils einer Zeile der Tabelle A entspricht

### Tabelle 174

Verbindungen der Formel I.1, in denen (X)ₘ 4-Nitro bedeutet, R² für Cyano steht und der Rest R³ für eine Verbindung jeweils einer Zeile der Tabelle A entspricht

### Tabelle 175

Verbindungen der Formel I.1, in denen (X)ₘ 5-Nitro bedeutet, R² für Wasserstoff steht und der Rest R³ für eine Verbindung jeweils einer Zeile der Tabelle A entspricht

### Tabelle 176

Verbindungen der Formel I.1, in denen (X)ₘ 5-Nitro bedeutet, R² für Methyl steht und der Rest R³ für eine Verbindung jeweils einer Zeile der Tabelle A entspricht

### Tabelle 177

Verbindungen der Formel I.1, in denen (X)ₘ 5-Nitro bedeutet, R² für Ethyl steht und der Rest R³ für eine Verbindung jeweils einer Zeile der Tabelle A entspricht

### Tabelle 178

Verbindungen der Formel I.1, in denen (X)ₘ 5-Nitro bedeutet, R² für n-Propyl steht und der Rest R³ für eine Verbindung jeweils einer Zeile der Tabelle A entspricht

### Tabelle 179

Verbindungen der Formel I.1, in denen (X)ₘ 5-Nitro bedeutet, R² für iso-Propyl steht und der Rest R³ für eine Verbindung jeweils einer Zeile der Tabelle A entspricht

### Tabelle 180

Verbindungen der Formel I.1, in denen (X)ₘ 5-Nitro bedeutet, R² für Cyano steht und der Rest R³ für eine Verbindung jeweils einer Zeile der Tabelle A entspricht

### Tabelle 181

Verbindungen der Formel I.2, in denen m Null bedeutet, R² für Wasserstoff steht und der Rest R³ für eine Verbindung jeweils einer Zeile der Tabelle A entspricht

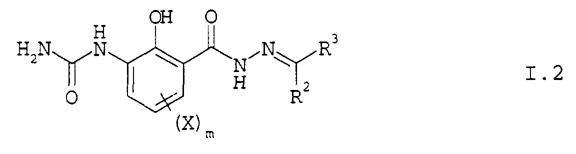

### Tabelle 182

Verbindungen der Formel I.2, in denen m Null bedeutet, R² für Methyl steht und der Rest R³ für eine Verbindung jeweils einer Zeile der Tabelle A entspricht

### Tabelle 183

Verbindungen der Formel I.2, in denen m Null bedeutet, R² für Ethyl steht und der Rest R³ für eine Verbindung jeweils einer Zeile der Tabelle A entspricht

### Tabelle 184

Verbindungen der Formel I.2, in denen m Null bedeutet, R² für n-Propyl steht und der Rest R³ für eine Verbindung jeweils einer Zeile der Tabelle A entspricht

### Tabelle 185

Verbindungen der Formel I.2, in denen m Null bedeutet, R² für iso-Propyl steht und der Rest R³ für eine Verbindung jeweils einer Zeile der Tabelle A entspricht

### Tabelle 186

Verbindungen der Formel I.2, in denen m Null bedeutet, R² für Cyano steht und der Rest R³ für eine Verbindung jeweils einer Zeile der Tabelle A entspricht

### Tabelle 187

Verbindungen der Formel I.2, in denen (X)ₘ 3-Chlor bedeutet, R² für Wasserstoff steht und der Rest R³ für eine Verbindung jeweils einer Zeile der Tabelle A entspricht

### Tabelle 188

Verbindungen der Formel I.2, in denen (X)ₘ 3-Chlor bedeutet, R² für Methyl steht und der Rest R³ für eine Verbindung jeweils einer Zeile der Tabelle A entspricht

### Tabelle 189

Verbindungen der Formel I.2, in denen (X)ₘ 3-Chlor bedeutet, R² für Ethyl steht und der Rest R³ für eine Verbindung jeweils einer Zeile der Tabelle A entspricht

### Tabelle 190

Verbindungen der Formel I.2, in denen (X)ₘ 3-Chlor bedeutet, R² für n-Propyl steht und der Rest R³ für eine Verbindung jeweils einer Zeile der Tabelle A entspricht

### Tabelle 191

Verbindungen der Formel I.2, in denen (X)ₘ 3-Chlor bedeutet, R² für iso-Propyl steht und der Rest R³ für eine Verbindung jeweils einer Zeile der Tabelle A entspricht

### Tabelle 192

Verbindungen der Formel I.2, in denen (X)ₘ 3-Chlor bedeutet, R² für Cyano steht und der Rest R³ für eine Verbindung jeweils einer Zeile der Tabelle A entspricht

### Tabelle 193

Verbindungen der Formel I.2, in denen (X)ₘ 4-Chlor bedeutet, R² für Wasserstoff steht und der Rest R³ für eine Verbindung jeweils einer Zeile der Tabelle A entspricht

### Tabelle 194

Verbindungen der Formel I.2, in denen (X)ₘ 4-Chlor bedeutet, R² für Methyl steht und der Rest R³ für eine Verbindung jeweils einer Zeile der Tabelle A entspricht

### Tabelle 195

Verbindungen der Formel I.2, in denen (X)ₘ 4-Chlor bedeutet, R² für Ethyl steht und der Rest R³ für eine Verbindung jeweils einer Zeile der Tabelle A entspricht

### Tabelle 196

Verbindungen der Formel I.2, in denen (X)ₘ 4-Chlor bedeutet, R² für n-Propyl steht und der Rest R³ für eine Verbindung jeweils einer Zeile der Tabelle A entspricht

### Tabelle 197

Verbindungen der Formel I.2, in denen (X)ₘ 4-Chlor bedeutet, R² für iso-Propyl steht und der Rest R³ für eine Verbindung jeweils einer Zeile der Tabelle A entspricht

### Tabelle 198

Verbindungen der Formel I.2, in denen (X)ₘ 4-Chlor bedeutet, R² für Cyano steht und der Rest R³ für eine Verbindung jeweils einer Zeile der Tabelle A entspricht

### Tabelle 199

Verbindungen der Formel I.2, in denen (X)ₘ 5-Chlor bedeutet, R² für Wasserstoff steht und der Rest R³ für eine Verbindung jeweils einer Zeile der Tabelle A entspricht

### Tabelle 200

Verbindungen der Formel I.2, in denen (X)ₘ 5-Chlor bedeutet, R² für Methyl steht und der Rest R³ für eine Verbindung jeweils einer Zeile der Tabelle A entspricht

### Tabelle 201

Verbindungen der Formel I.2, in denen (X)ₘ 5-Chlor bedeutet, R² für Ethyl steht und der Rest R³ für eine Verbindung jeweils einer Zeile der Tabelle A entspricht

### Tabelle 202

Verbindungen der Formel I.2, in denen (X)ₘ 5-Chlor bedeutet, R² für n-Propyl steht und der Rest R³ für eine Verbindung jeweils einer Zeile der Tabelle A entspricht

### Tabelle 203

Verbindungen der Formel I.2, in denen (X)ₘ 5-Chlor bedeutet, R² für iso-Propyl steht und der Rest R³ für eine Verbindung jeweils einer Zeile der Tabelle A entspricht

### Tabelle 204

Verbindungen der Formel I.2, in denen (X)ₘ 5-Chlor bedeutet, R² für Cyano steht und der Rest R³ für eine Verbindung jeweils einer Zeile der Tabelle A entspricht

### Tabelle 205

Verbindungen der Formel I.2, in denen (X)ₘ 3-Brom bedeutet, R² für Wasserstoff steht und der Rest R³ für eine Verbindung jeweils einer Zeile der Tabelle A entspricht

### Tabelle 206

Verbindungen der Formel I.2, in denen (X)ₘ 3-Brom bedeutet, R² für Methyl steht und der Rest R³ für eine Verbindung jeweils einer Zeile der Tabelle A entspricht

### Tabelle 207

Verbindungen der Formel I.2, in denen (X)ₘ 3-Brom bedeutet, R² für Ethyl steht und der Rest R³ für eine Verbindung jeweils einer Zeile der Tabelle A entspricht

### Tabelle 208

Verbindungen der Formel I.2, in denen (X)ₘ 3-Brom bedeutet, R² für n-Propyl steht und der Rest R³ für eine Verbindung jeweils einer Zeile der Tabelle A entspricht

### Tabelle 209

Verbindungen der Formel I.2, in denen (X)ₘ 3-Brom bedeutet, R² für iso-Propyl steht und der Rest R³ für eine Verbindung jeweils einer Zeile der Tabelle A entspricht

### Tabelle 210

Verbindungen der Formel I.2, in denen (X)ₘ 3-Brom bedeutet, R² für Cyano steht und der Rest R³ für eine Verbindung jeweils einer Zeile der Tabelle A entspricht

### Tabelle 211

Verbindungen der Formel I.2, in denen (X)ₘ 4-Brom bedeutet, R² für Wasserstoff steht und der Rest R³ für eine Verbindung jeweils einer Zeile der Tabelle A entspricht

### Tabelle 212

Verbindungen der Formel I.2, in denen (X)ₘ 4-Brom bedeutet, R² für Methyl steht und der Rest R³ für eine Verbindung jeweils einer Zeile der Tabelle A entspricht

### Tabelle 213

Verbindungen der Formel I.2, in denen (X)ₘ 4-Brom bedeutet, R² für Ethyl steht und der Rest R³ für eine Verbindung jeweils einer Zeile der Tabelle A entspricht

### Tabelle 214

Verbindungen der Formel I.2, in denen (X)ₘ 4-Brom bedeutet, R² für n-Propyl steht und der Rest R³ für eine Verbindung jeweils einer Zeile der Tabelle A entspricht

### Tabelle 215

Verbindungen der Formel I.2, in denen (X)ₘ 4-Brom bedeutet, R² für iso-Propyl steht und der Rest R³ für eine Verbindung jeweils einer Zeile der Tabelle A entspricht

### Tabelle 216

Verbindungen der Formel I.2, in denen (X)ₘ 4-Brom bedeutet, R² für Cyano steht und der Rest R³ für eine Verbindung jeweils einer Zeile der Tabelle A entspricht

### Tabelle 217

Verbindungen der Formel I.2, in denen (X)ₘ 5-Brom bedeutet, R² für Wasserstoff steht und der Rest R³ für eine Verbindung jeweils einer Zeile der Tabelle A entspricht

### Tabelle 218

Verbindungen der Formel I.2, in denen (X)ₘ 5-Brom bedeutet, R² für Methyl steht und der Rest R³ für eine Verbindung jeweils einer Zeile der Tabelle A entspricht

### Tabelle 219

Verbindungen der Formel I.2, in denen (X)ₘ 5-Brom bedeutet, R² für Ethyl steht und der Rest R³ für eine Verbindung jeweils einer Zeile der Tabelle A entspricht

### Tabelle 220

Verbindungen der Formel I.2, in denen (X)ₘ 5-Brom bedeutet, R² für n-Propyl steht und der Rest R³ für eine Verbindung jeweils einer Zeile der Tabelle A entspricht

### Tabelle 221

Verbindungen der Formel I.2, in denen (X)ₘ 5-Brom bedeutet, R² für iso-Propyl steht und der Rest R³ für eine Verbindung jeweils einer Zeile der Tabelle A entspricht

### Tabelle 222

Verbindungen der Formel I.2, in denen (X)ₘ 5-Brom bedeutet, R² für Cyano steht und der Rest R³ für eine Verbindung jeweils einer Zeile der Tabelle A entspricht

### Tabelle 223

Verbindungen der Formel I.2, in denen (X)ₘ 3-Nitro bedeutet, R² für Wasserstoff steht und der Rest R³ für eine Verbindung jeweils einer Zeile der Tabelle A entspricht

### Tabelle 224

Verbindungen der Formel I.2, in denen (X)ₘ 3-Nitro bedeutet, R² für Methyl steht und der Rest R³ für eine Verbindung jeweils einer Zeile der Tabelle A entspricht

### Tabelle 225

Verbindungen der Formel I.2, in denen (X)ₘ 3-Nitro bedeutet, R² für Ethyl steht und der Rest R³ für eine Verbindung jeweils einer Zeile der Tabelle A entspricht

### Tabelle 226

Verbindungen der Formel I.2, in denen (X)ₘ 3-Nitro bedeutet, R² für n-Propyl steht und der Rest R³ für eine Verbindung jeweils einer Zeile der Tabelle A entspricht

### Tabelle 227

Verbindungen der Formel I.2, in denen (X)ₘ 3-Nitro bedeutet, R² für iso-Propyl steht und der Rest R³ für eine Verbindung jeweils einer Zeile der Tabelle A entspricht

### Tabelle 228

Verbindungen der Formel I.2, in denen (X)ₘ 3-Nitro bedeutet, R² für Cyano steht und der Rest R³ für eine Verbindung jeweils einer Zeile der Tabelle A entspricht

### Tabelle 229

Verbindungen der Formel I.2, in denen (X)ₘ 4-Nitro bedeutet, R² für Wasserstoff steht und der Rest R³ für eine Verbindung jeweils einer Zeile der Tabelle A entspricht

### Tabelle 230

Verbindungen der Formel I.2, in denen (X)ₘ 4-Nitro bedeutet, R² für Methyl steht und der Rest R³ für eine Verbindung jeweils einer Zeile der Tabelle A entspricht

### Tabelle 231

Verbindungen der Formel I.2, in denen (X)ₘ 4-Nitro bedeutet, R² für Ethyl steht und der Rest R³ für eine Verbindung jeweils einer Zeile der Tabelle A entspricht

### Tabelle 232

Verbindungen der Formel I.2, in denen (X)ₘ 4-Nitro bedeutet, R² für n-Propyl steht und der Rest R³ für eine Verbindung jeweils einer Zeile der Tabelle A entspricht

### Tabelle 233

Verbindungen der Formel I.2, in denen (X)ₘ 4-Nitro bedeutet, R² für iso-Propyl steht und der Rest R³ für eine Verbindung jeweils einer Zeile der Tabelle A entspricht

### Tabelle 234

Verbindungen der Formel I.2, in denen (X)ₘ 4-Nitro bedeutet, R² für Cyano steht und der Rest R³ für eine Verbindung jeweils einer Zeile der Tabelle A entspricht

### Tabelle 235

Verbindungen der Formel I.2, in denen (X)ₘ 5-Nitro bedeutet, R² für Wasserstoff steht und der Rest R³ für eine Verbindung jeweils einer Zeile der Tabelle A entspricht

### Tabelle 236

Verbindungen der Formel I.2, in denen (X)ₘ 5-Nitro bedeutet, R² für Methyl steht und der Rest R³ für eine Verbindung jeweils einer Zeile der Tabelle A entspricht

### Tabelle 237

Verbindungen der Formel I.2, in denen (X)ₘ 5-Nitro bedeutet, R² für Ethyl steht und der Rest R³ für eine Verbindung jeweils einer Zeile der Tabelle A entspricht

### Tabelle 238

Verbindungen der Formel I.2, in denen (X)ₘ 5-Nitro bedeutet, R² für n-Propyl steht und der Rest R³ für eine Verbindung jeweils einer Zeile der Tabelle A entspricht

### Tabelle 239

Verbindungen der Formel I.2, in denen (X)ₘ 5-Nitro bedeutet, R² für iso-Propyl steht und der Rest R³ für eine Verbindung jeweils einer Zeile der Tabelle A entspricht

### Tabelle 240

Verbindungen der Formel I.2, in denen (X)ₘ 5-Nitro bedeutet, R² für Cyano steht und der Rest R³ für eine Verbindung jeweils einer Zeile der Tabelle A entspricht

### Tabelle 241

Verbindungen der Formel I.3, in denen m Null bedeutet, R² für Wasserstoff steht und der Rest R³ für eine Verbindung jeweils einer Zeile der Tabelle A entspricht

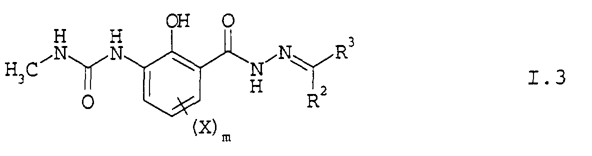

### Tabelle 242

Verbindungen der Formel I.3, in denen m Null bedeutet, R² für Methyl steht und der Rest R³ für eine Verbindung jeweils einer Zeile der Tabelle A entspricht

### Tabelle 243

Verbindungen der Formel I.3, in denen m Null bedeutet, R² für Ethyl steht und der Rest R³ für eine Verbindung jeweils einer Zeile der Tabelle A entspricht

### Tabelle 244

Verbindungen der Formel I.3, in denen m Null bedeutet, R² für n-Propyl steht und der Rest R³ für eine Verbindung jeweils einer Zeile der Tabelle A entspricht

### Tabelle 245

Verbindungen der Formel I.3, in denen m Null bedeutet, R² für iso-Propyl steht und der Rest R³ für eine Verbindung jeweils einer Zeile der Tabelle A entspricht

### Tabelle 246

Verbindungen der Formel I.3, in denen m Null bedeutet, R² für Cyano steht und der Rest R³ für eine Verbindung jeweils einer Zeile der Tabelle A entspricht

### Tabelle 247

Verbindungen der Formel I.3, in denen (X)ₘ 3-Chlor bedeutet, R² für Wasserstoff steht und der Rest R³ für eine Verbindung jeweils einer Zeile der Tabelle A entspricht

### Tabelle 248

Verbindungen der Formel I.3, in denen (X)ₘ 3-Chlor bedeutet, R² für Methyl steht und der Rest R³ für eine Verbindung jeweils einer Zeile der Tabelle A entspricht

### Tabelle 249

Verbindungen der Formel I.3, in denen (X)ₘ 3-Chlor bedeutet, R² für Ethyl steht und der Rest R³ für eine Verbindung jeweils einer Zeile der Tabelle A entspricht

### Tabelle 250

Verbindungen der Formel I.3, in denen (X)ₘ 3-Chlor bedeutet, R² für n-Propyl steht und der Rest R³ für eine Verbindung jeweils einer Zeile der Tabelle A entspricht

### Tabelle 251

Verbindungen der Formel I.3, in denen (X)ₘ 3-Chlor bedeutet, R² für iso-Propyl steht und der Rest R³ für eine Verbindung jeweils einer Zeile der Tabelle A entspricht

### Tabelle 252

Verbindungen der Formel I.3, in denen (X)ₘ 3-Chlor bedeutet, R² für Cyano steht und der Rest R³ für eine Verbindung jeweils einer Zeile der Tabelle A entspricht

### Tabelle 253

Verbindungen der Formel I.3, in denen (X)ₘ 4-Chlor bedeutet, R² für Wasserstoff steht und der Rest R³ für eine Verbindung jeweils einer Zeile der Tabelle A entspricht

### Tabelle 254

Verbindungen der Formel I.3, in denen (X)ₘ 4-Chlor bedeutet, R² für Methyl steht und der Rest R³ für eine Verbindung jeweils einer Zeile der Tabelle A entspricht

### Tabelle 255

Verbindungen der Formel I.3, in denen (X)ₘ 4-Chlor bedeutet, R² für Ethyl steht und der Rest R³ für eine Verbindung jeweils einer Zeile der Tabelle A entspricht

### Tabelle 256

Verbindungen der Formel I.3, in denen (X)ₘ 4-Chlor bedeutet, R² für n-Propyl steht und der Rest R³ für eine Verbindung jeweils einer Zeile der Tabelle A entspricht

### Tabelle 257

Verbindungen der Formel I.3, in denen (X)ₘ 4-Chlor bedeutet, R² für iso-Propyl steht und der Rest R³ für eine Verbindung jeweils einer Zeile der Tabelle A entspricht

### Tabelle 258

Verbindungen der Formel I.3, in denen (X)ₘ 4-Chlor bedeutet, R² für Cyano steht und der Rest R³ für eine Verbindung jeweils einer Zeile der Tabelle A entspricht

### Tabelle 259

Verbindungen der Formel I.3, in denen (X)ₘ 5-Chlor bedeutet, R² für Wasserstoff steht und der Rest R³ für eine Verbindung jeweils einer Zeile der Tabelle A entspricht

### Tabelle 260

Verbindungen der Formel I.3, in denen (X)ₘ 5-Chlor bedeutet, R² für Methyl steht und der Rest R³ für eine Verbindung jeweils einer Zeile der Tabelle A entspricht

### Tabelle 261

Verbindungen der Formel I.3, in denen (X)ₘ 5-Chlor bedeutet, R² für Ethyl steht und der Rest R³ für eine Verbindung jeweils einer Zeile der Tabelle A entspricht

### Tabelle 262

Verbindungen der Formel I.3, in denen (X)ₘ 5-Chlor bedeutet, R² für n-Propyl steht und der Rest R³ für eine Verbindung jeweils einer Zeile der Tabelle A entspricht

### Tabelle 263

Verbindungen der Formel I.3, in denen (X)ₘ 5-Chlor bedeutet, R² für iso-Propyl steht und der Rest R³ für eine Verbindung jeweils einer Zeile der Tabelle A entspricht

### Tabelle 264

Verbindungen der Formel I.3, in denen (X)ₘ 5-Chlor bedeutet, R² für Cyano steht und der Rest R³ für eine Verbindung jeweils einer Zeile der Tabelle A entspricht

### Tabelle 265

Verbindungen der Formel I.3, in denen (X)ₘ 3-Brom bedeutet, R² für Wasserstoff steht und der Rest R³ für eine Verbindung jeweils einer Zeile der Tabelle A entspricht

### Tabelle 266

Verbindungen der Formel I.3, in denen (X)ₘ 3-Brom bedeutet, R² für Methyl steht und der Rest R³ für eine Verbindung jeweils einer Zeile der Tabelle A entspricht

### Tabelle 267

Verbindungen der Formel I.3, in denen (X)ₘ 3-Brom bedeutet, R² für Ethyl steht und der Rest R³ für eine Verbindung jeweils einer Zeile der Tabelle A entspricht

### Tabelle 268

Verbindungen der Formel I.3, in denen (X)ₘ 3-Brom bedeutet, R² für n-Propyl steht und der Rest R³ für eine Verbindung jeweils einer Zeile der Tabelle A entspricht

### Tabelle 269

Verbindungen der Formel I.3, in denen (X)ₘ 3-Brom bedeutet, R² für iso-Propyl steht und der Rest R³ für eine Verbindung jeweils einer Zeile der Tabelle A entspricht

### Tabelle 270

Verbindungen der Formel I.3, in denen (X)ₘ 3-Brom bedeutet, R² für Cyano steht und der Rest R³ für eine Verbindung jeweils einer Zeile der Tabelle A entspricht

### Tabelle 271

Verbindungen der Formel I.3, in denen (X)ₘ 4-Brom bedeutet, R² für Wasserstoff steht und der Rest R³ für eine Verbindung jeweils einer Zeile der Tabelle A entspricht

### Tabelle 272

Verbindungen der Formel I.3, in denen (X)ₘ 4-Brom bedeutet, R² für Methyl steht und der Rest R³ für eine Verbindung jeweils einer Zeile der Tabelle A entspricht

### Tabelle 273

Verbindungen der Formel I.3, in denen (X)ₘ 4-Brom bedeutet, R² für Ethyl steht und der Rest R³ für eine Verbindung jeweils einer Zeile der Tabelle A entspricht

### Tabelle 274

Verbindungen der Formel I.3, in denen (X)ₘ 4-Brom bedeutet, R² für n-Propyl steht und der Rest R³ für eine Verbindung jeweils einer Zeile der Tabelle A entspricht

### Tabelle 275

Verbindungen der Formel I.3, in denen (X)ₘ 4-Brom bedeutet, R² für iso-Propyl steht und der Rest R³ für eine Verbindung jeweils einer Zeile der Tabelle A entspricht

### Tabelle 276

Verbindungen der Formel I.3, in denen (X)ₘ 4-Brom bedeutet, R² für Cyano steht und der Rest R³ für eine Verbindung jeweils einer Zeile der Tabelle A entspricht

### Tabelle 277

Verbindungen der Formel I.3, in denen (X)ₘ 5-Brom bedeutet, R² für Wasserstoff steht und der Rest R³ für eine Verbindung jeweils einer Zeile der Tabelle A entspricht

### Tabelle 278

Verbindungen der Formel I.3, in denen (X)ₘ 5-Brom bedeutet, R² für Methyl steht und der Rest R³ für eine Verbindung jeweils einer Zeile der Tabelle A entspricht

### Tabelle 279

Verbindungen der Formel I.3, in denen (X)ₘ 5-Brom bedeutet, R² für Ethyl steht und der Rest R³ für eine Verbindung jeweils einer Zeile der Tabelle A entspricht

### Tabelle 280

Verbindungen der Formel I.3, in denen (X)ₘ 5-Brom bedeutet, R² für n-Propyl steht und der Rest R³ für eine Verbindung jeweils einer Zeile der Tabelle A entspricht

### Tabelle 281

Verbindungen der Formel I.3, in denen (X)ₘ 5-Brom bedeutet, R² für iso-Propyl steht und der Rest R³ für eine Verbindung jeweils einer Zeile der Tabelle A entspricht

### Tabelle 282

Verbindungen der Formel I.3, in denen (X)ₘ 5-Brom bedeutet, R² für Cyano steht und der Rest R³ für eine Verbindung jeweils einer Zeile der Tabelle A entspricht

### Tabelle 283

Verbindungen der Formel I.3, in denen (X)ₘ 3-Nitro bedeutet, R² für Wasserstoff steht und der Rest R³ für eine Verbindung jeweils einer Zeile der Tabelle A entspricht

### Tabelle 284

Verbindungen der Formel I.3, in denen (X)ₘ 3-Nitro bedeutet, R² für Methyl steht und der Rest R³ für eine Verbindung jeweils einer Zeile der Tabelle A entspricht

### Tabelle 285

Verbindungen der Formel I.3, in denen (X)ₘ 3-Nitro bedeutet, R² für Ethyl steht und der Rest R³ für eine Verbindung jeweils einer Zeile der Tabelle A entspricht

### Tabelle 286

Verbindungen der Formel I.3, in denen (X)ₘ 3-Nitro bedeutet, R² für n-Propyl steht und der Rest R³ für eine Verbindung jeweils einer Zeile der Tabelle A entspricht

### Tabelle 287

Verbindungen der Formel I.3, in denen (X)ₘ 3-Nitro bedeutet, R² für iso-Propyl steht und der Rest R³ für eine Verbindung jeweils einer Zeile der Tabelle A entspricht

### Tabelle 288

Verbindungen der Formel I.3, in denen (X)ₘ 3-Nitro bedeutet, R² für Cyano steht und der Rest R³ für eine Verbindung jeweils einer Zeile der Tabelle A entspricht

### Tabelle 289

Verbindungen der Formel I.3, in denen (X)ₘ 4-Nitro bedeutet, R² für Wasserstoff steht und der Rest R³ für eine Verbindung jeweils einer Zeile der Tabelle A entspricht

### Tabelle 290

Verbindungen der Formel I.3, in denen (X)ₘ 4-Nitro bedeutet, R² für Methyl steht und der Rest R³ für eine Verbindung jeweils einer Zeile der Tabelle A entspricht

### Tabelle 291

Verbindungen der Formel I.3, in denen (X)ₘ 4-Nitro bedeutet, R² für Ethyl steht und der Rest R³ für eine Verbindung jeweils einer Zeile der Tabelle A entspricht

### Tabelle 292

Verbindungen der Formel I.3, in denen (X)ₘ 4-Nitro bedeutet, R² für n-Propyl steht und der Rest R³ für eine Verbindung jeweils einer Zeile der Tabelle A entspricht

### Tabelle 293

Verbindungen der Formel I.3, in denen (X)ₘ 4-Nitro bedeutet, R² für iso-Propyl steht und der Rest R³ für eine Verbindung jeweils einer Zeile der Tabelle A entspricht

### Tabelle 294

Verbindungen der Formel I.3, in denen (X)ₘ 4-Nitro bedeutet, R² für Cyano steht und der Rest R³ für eine Verbindung jeweils einer Zeile der Tabelle A entspricht

### Tabelle 295

Verbindungen der Formel I.3, in denen (X)ₘ 5-Nitro bedeutet, R² für Wasserstoff steht und der Rest R³ für eine Verbindung jeweils einer Zeile der Tabelle A entspricht

### Tabelle 296

Verbindungen der Formel I.3, in denen (X)ₘ 5-Nitro bedeutet, R² für Methyl steht und der Rest R³ für eine Verbindung jeweils einer Zeile der Tabelle A entspricht

### Tabelle 297

Verbindungen der Formel I.3, in denen (X)ₘ 5-Nitro bedeutet, R² für Ethyl steht und der Rest R³ für eine Verbindung jeweils einer Zeile der Tabelle A entspricht

### Tabelle 298

Verbindungen der Formel I.3, in denen (X)ₘ 5-Nitro bedeutet, R² für n-Propyl steht und der Rest R³ für eine Verbindung jeweils einer Zeile der Tabelle A entspricht

### Tabelle 299

Verbindungen der Formel I.3, in denen (X)ₘ 5-Nitro bedeutet, R² für iso-Propyl steht und der Rest R³ für eine Verbindung jeweils einer Zeile der Tabelle A entspricht

### Tabelle 300

Verbindungen der Formel I.3, in denen (X)ₘ 5-Nitro bedeutet, R² für Cyano steht und der Rest R³ für eine Verbindung jeweils einer Zeile der Tabelle A entspricht

### Tabelle 301

Verbindungen der Formel I.5, in denen m Null bedeutet, R² für Wasserstoff steht und der Rest R³ für eine Verbindung jeweils einer Zeile der Tabelle A entspricht

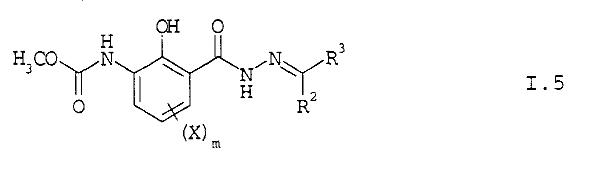

### Tabelle 302

Verbindungen der Formel I.5, in denen m Null bedeutet, R² für Methyl steht und der Rest R³ für eine Verbindung jeweils einer Zeile der Tabelle A entspricht

### Tabelle 303

Verbindungen der Formel I.5, in denen m Null bedeutet, R² für Ethyl steht und der Rest R³ für eine Verbindung jeweils einer Zeile der Tabelle A entspricht

### Tabelle 304

Verbindungen der Formel I.5, in denen m Null bedeutet, R² für n-Propyl steht und der Rest R³ für eine Verbindung jeweils einer Zeile der Tabelle A entspricht

### Tabelle 305

Verbindungen der Formel I.5, in denen m Null bedeutet, R² für iso-Propyl steht und der Rest R³ für eine Verbindung jeweils einer Zeile der Tabelle A entspricht

### Tabelle 306

Verbindungen der Formel I.5, in denen m Null bedeutet, R² für Cyano steht und der Rest R³ für eine Verbindung jeweils einer Zeile der Tabelle A entspricht

### Tabelle 307

Verbindungen der Formel I.5, in denen (X)ₘ 3-Chlor bedeutet, R² für Wasserstoff steht und der Rest R³ für eine Verbindung jeweils einer Zeile der Tabelle A entspricht

### Tabelle 308

Verbindungen der Formel I.5, in denen (X)ₘ 3-Chlor bedeutet, R² für Methyl steht und der Rest R³ für eine Verbindung jeweils einer Zeile der Tabelle A entspricht

### Tabelle 309

Verbindungen der Formel I.5, in denen (X)ₘ 3-Chlor bedeutet, R² für Ethyl steht und der Rest R³ für eine Verbindung jeweils einer Zeile der Tabelle A entspricht

### Tabelle 310

Verbindungen der Formel I.5, in denen (X)ₘ 3-Chlor bedeutet, R² für n-Propyl steht und der Rest R³ für eine Verbindung jeweils einer Zeile der Tabelle A entspricht

### Tabelle 311

Verbindungen der Formel I.5, in denen (X)ₘ 3-Chlor bedeutet, R² für iso-Propyl steht und der Rest R³ für eine Verbindung jeweils einer Zeile der Tabelle A entspricht

### Tabelle 312

Verbindungen der Formel I.5, in denen (X)ₘ 3-Chlor bedeutet, R² für Cyano steht und der Rest R³ für eine Verbindung jeweils einer Zeile der Tabelle A entspricht

### Tabelle 313

Verbindungen der Formel I.5, in denen (X)ₘ 4-Chlor bedeutet, R² für Wasserstoff steht und der Rest R³ für eine Verbindung jeweils einer Zeile der Tabelle A entspricht

### Tabelle 314

Verbindungen der Formel I.5, in denen (X)ₘ 4-Chlor bedeutet, R² für Methyl steht und der Rest R³ für eine Verbindung jeweils einer Zeile der Tabelle A entspricht

### Tabelle 315

Verbindungen der Formel I.5, in denen (X)ₘ 4-Chlor bedeutet, R² für Ethyl steht und der Rest R³ für eine Verbindung jeweils einer Zeile der Tabelle A entspricht

### Tabelle 316

Verbindungen der Formel I.5, in denen (X)ₘ 4-Chlor bedeutet, R² für n-Propyl steht und der Rest R³ für eine Verbindung jeweils einer Zeile der Tabelle A entspricht

### Tabelle 317

Verbindungen der Formel I.5, in denen (X)ₘ 4-Chlor bedeutet, R² für iso-Propyl steht und der Rest R³ für eine Verbindung jeweils einer Zeile der Tabelle A entspricht

### Tabelle 318

Verbindungen der Formel I.5, in denen (X)ₘ 4-Chlor bedeutet, R² für Cyano steht und der Rest R³ für eine Verbindung jeweils einer Zeile der Tabelle A entspricht

### Tabelle 319

Verbindungen der Formel I.5, in denen (X)ₘ 5-Chlor bedeutet, R² für Wasserstoff steht und der Rest R³ für eine Verbindung jeweils einer Zeile der Tabelle A entspricht

### Tabelle 320

Verbindungen der Formel I.5, in denen (X)ₘ 5-Chlor bedeutet, R² für Methyl steht und der Rest R³ für eine Verbindung jeweils einer Zeile der Tabelle A entspricht

### Tabelle 321

Verbindungen der Formel I.5, in denen (X)ₘ 5-Chlor bedeutet, R² für Ethyl steht und der Rest R³ für eine Verbindung jeweils einer Zeile der Tabelle A entspricht

### Tabelle 322

Verbindungen der Formel I.5, in denen (X)ₘ 5-Chlor bedeutet, R² für n-Propyl steht und der Rest R³ für eine Verbindung jeweils einer Zeile der Tabelle A entspricht

### Tabelle 323

Verbindungen der Formel I.5, in denen (X)ₘ 5-Chlor bedeutet, R² für iso-Propyl steht und der Rest R³ für eine Verbindung jeweils einer Zeile der Tabelle A entspricht

### Tabelle 324

Verbindungen der Formel I.5, in denen (X)ₘ 5-Chlor bedeutet, R² für Cyano steht und der Rest R³ für eine Verbindung jeweils einer Zeile der Tabelle A entspricht

### Tabelle 325

Verbindungen der Formel I.5, in denen (X)ₘ 3-Brom bedeutet, R² für Wasserstoff steht und der Rest R³ für eine Verbindung jeweils einer Zeile der Tabelle A entspricht

### Tabelle 326

Verbindungen der Formel I.5, in denen (X)ₘ 3-Brom bedeutet, R² für Methyl steht und der Rest R³ für eine Verbindung jeweils einer Zeile der Tabelle A entspricht

### Tabelle 327

Verbindungen der Formel I.5, in denen (X)ₘ 3-Brom bedeutet, R² für Ethyl steht und der Rest R³ für eine Verbindung jeweils einer Zeile der Tabelle A entspricht

### Tabelle 328

Verbindungen der Formel I.5, in denen (X)ₘ 3-Brom bedeutet, R² für n-Propyl steht und der Rest R³ für eine Verbindung jeweils einer Zeile der Tabelle A entspricht

### Tabelle 329

Verbindungen der Formel I.5, in denen (X)ₘ 3-Brom bedeutet, R² für iso-Propyl steht und der Rest R³ für eine Verbindung jeweils einer Zeile der Tabelle A entspricht

### Tabelle 330

Verbindungen der Formel I.5, in denen (X)ₘ 3-Brom bedeutet, R² für Cyano steht und der Rest R³ für eine Verbindung jeweils einer Zeile der Tabelle A entspricht

### Tabelle 331

Verbindungen der Formel I.5, in denen (X)ₘ 4-Brom bedeutet, R² für Wasserstoff steht und der Rest R³ für eine Verbindung jeweils einer Zeile der Tabelle A entspricht

### Tabelle 332

Verbindungen der Formel I.5, in denen (X)ₘ 4-Brom bedeutet, R² für Methyl steht und der Rest R³ für eine Verbindung jeweils einer Zeile der Tabelle A entspricht

### Tabelle 333

Verbindungen der Formel I.5, in denen (X)ₘ 4-Brom bedeutet, R² für Ethyl steht und der Rest R³ für eine Verbindung jeweils einer Zeile der Tabelle A entspricht

### Tabelle 334

Verbindungen der Formel I.5, in denen (X)ₘ 4-Brom bedeutet, R² für n-Propyl steht und der Rest R³ für eine Verbindung jeweils einer Zeile der Tabelle A entspricht

### Tabelle 335

Verbindungen der Formel I.5, in denen (X)ₘ 4-Brom bedeutet, R² für iso-Propyl steht und der Rest R³ für eine Verbindung jeweils einer Zeile der Tabelle A entspricht

### Tabelle 336

Verbindungen der Formel I.5, in denen (X)ₘ 4-Brom bedeutet, R² für Cyano steht und der Rest R³ für eine Verbindung jeweils einer Zeile der Tabelle A entspricht

### Tabelle 337

Verbindungen der Formel I.5, in denen (X)ₘ 5-Brom bedeutet, R² für Wasserstoff steht und der Rest R³ für eine Verbindung jeweils einer Zeile der Tabelle A entspricht

### Tabelle 338

Verbindungen der Formel I.5, in denen (X)ₘ 5-Brom bedeutet, R² für Methyl steht und der Rest R³ für eine Verbindung jeweils einer Zeile der Tabelle A entspricht

### Tabelle 339

Verbindungen der Formel I.5, in denen (X)ₘ 5-Brom bedeutet, R² für Ethyl steht und der Rest R³ für eine Verbindung jeweils einer Zeile der Tabelle A entspricht

### Tabelle 340

Verbindungen der Formel I.5, in denen (X)ₘ 5-Brom bedeutet, R² für n-Propyl steht und der Rest R³ für eine Verbindung jeweils einer Zeile der Tabelle A entspricht

### Tabelle 341

Verbindungen der Formel I.5, in denen (X)ₘ 5-Brom bedeutet, R² für iso-Propyl steht und der Rest R³ für eine Verbindung jeweils einer Zeile der Tabelle A entspricht

### Tabelle 342

Verbindungen der Formel I.5, in denen (X)ₘ 5-Brom bedeutet, R² für Cyano steht und der Rest R³ für eine Verbindung jeweils einer Zeile der Tabelle A entspricht

### Tabelle 343

Verbindungen der Formel I.5, in denen (X)ₘ 3-Nitro bedeutet, R² für Wasserstoff steht und der Rest R³ für eine Verbindung jeweils einer Zeile der Tabelle A entspricht

### Tabelle 344

Verbindungen der Formel I.5, in denen (X)ₘ 3-Nitro bedeutet, R² für Methyl steht und der Rest R³ für eine Verbindung jeweils einer Zeile der Tabelle A entspricht

### Tabelle 345

Verbindungen der Formel I.5, in denen (X)ₘ 3-Nitro bedeutet, R² für Ethyl steht und der Rest R³ für eine Verbindung jeweils einer Zeile der Tabelle A entspricht

### Tabelle 346

Verbindungen der Formel I.5, in denen (X)ₘ 3-Nitro bedeutet, R² für n-Propyl steht und der Rest R³ für eine Verbindung jeweils einer Zeile der Tabelle A entspricht

### Tabelle 347

Verbindungen der Formel I.5, in denen (X)ₘ 3-Nitro bedeutet, R² für iso-Propyl steht und der Rest R³ für eine Verbindung jeweils einer Zeile der Tabelle A entspricht

### Tabelle 348

Verbindungen der Formel I.5, in denen (X)ₘ 3-Nitro bedeutet, R² für Cyano steht und der Rest R³ für eine Verbindung jeweils einer Zeile der Tabelle A entspricht

### Tabelle 349

Verbindungen der Formel I.5, in denen (X)ₘ 4-Nitro bedeutet, R² für Wasserstoff steht und der Rest R³ für eine Verbindung jeweils einer Zeile der Tabelle A entspricht

### Tabelle 350

Verbindungen der Formel I.5, in denen (X)ₘ 4-Nitro bedeutet, R² für Methyl steht und der Rest R³ für eine Verbindung jeweils einer Zeile der Tabelle A entspricht

### Tabelle 351

Verbindungen der Formel I.5, in denen (X)ₘ 4-Nitro bedeutet, R² für Ethyl steht und der Rest R³ für eine Verbindung jeweils einer Zeile der Tabelle A entspricht

### Tabelle 352

Verbindungen der Formel I.5, in denen (X)ₘ 4-Nitro bedeutet, R² für n-Propyl steht und der Rest R³ für eine Verbindung jeweils einer Zeile der Tabelle A entspricht

### Tabelle 353

Verbindungen der Formel I.5, in denen (X)ₘ 4-Nitro bedeutet, R² für iso-Propyl steht und der Rest R³ für eine Verbindung jeweils einer Zeile der Tabelle A entspricht

### Tabelle 354

Verbindungen der Formel I.5, in denen (X)ₘ 4-Nitro bedeutet, R² für Cyano steht und der Rest R³ für eine Verbindung jeweils einer Zeile der Tabelle A entspricht

### Tabelle 355

Verbindungen der Formel I.5, in denen (X)ₘ 5-Nitro bedeutet, R² für Wasserstoff steht und der Rest R³ für eine Verbindung jeweils einer Zeile der Tabelle A entspricht

### Tabelle 356

Verbindungen der Formel I.5, in denen (X)ₘ 5-Nitro bedeutet, R² für Methyl steht und der Rest R³ für eine Verbindung jeweils einer Zeile der Tabelle A entspricht

### Tabelle 357

Verbindungen der Formel I.5, in denen (X)ₘ 5-Nitro bedeutet, R² für Ethyl steht und der Rest R³ für eine Verbindung jeweils einer Zeile der Tabelle A entspricht

### Tabelle 358

Verbindungen der Formel I.5, in denen (X)ₘ 5-Nitro bedeutet, R² für n-Propyl steht und der Rest R³ für eine Verbindung jeweils einer Zeile der Tabelle A entspricht

### Tabelle 359

Verbindungen der Formel I.5, in denen (X)ₘ 5-Nitro bedeutet, R² für iso-Propyl steht und der Rest R³ für eine Verbindung jeweils einer Zeile der Tabelle A entspricht

### Tabelle 360

Verbindungen der Formel I.5, in denen (X)ₘ 5-Nitro bedeutet, R² für Cyano steht und der Rest R³ für eine Verbindung jeweils einer Zeile der Tabelle A entspricht

### Tabelle 361

Verbindungen der Formel I.6a, in denen m Null bedeutet, R² für Wasserstoff steht und der Rest R³ für eine Verbindung jeweils einer Zeile der Tabelle A entspricht

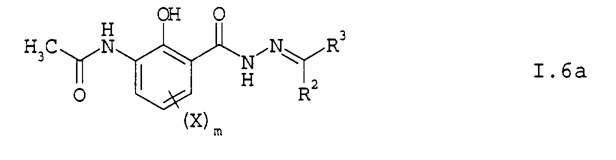

### Tabelle 362

Verbindungen der Formel I.6a, in denen m Null bedeutet, R² für Methyl steht und der Rest R³ für eine Verbindung jeweils einer Zeile der Tabelle A entspricht

### Tabelle 363

Verbindungen der Formel I.6a, in denen m Null bedeutet, R² für Ethyl steht und der Rest R³ für eine Verbindung jeweils einer Zeile der Tabelle A entspricht

### Tabelle 364

Verbindungen der Formel I.6a, in denen m Null bedeutet, R² für n-Propyl steht und der Rest R³ für eine Verbindung jeweils einer Zeile der Tabelle A entspricht

### Tabelle 365

Verbindungen der Formel I.6a, in denen m Null bedeutet, R² für iso-Propyl steht und der Rest R³ für eine Verbindung jeweils einer Zeile der Tabelle A entspricht

### Tabelle 366

Verbindungen der Formel I.6a, in denen m Null bedeutet, R² für Cyano steht und der Rest R³ für eine Verbindung jeweils einer Zeile der Tabelle A entspricht

### Tabelle 367

Verbindungen der Formel I.6a, in denen (X)ₘ 3-Chlor bedeutet, R² für Wasserstoff steht und der Rest R³ für eine Verbindung jeweils einer Zeile der Tabelle A entspricht

### Tabelle 368

Verbindungen der Formel I.6a, in denen (X)ₘ 3-Chlor bedeutet, R² für Methyl steht und der Rest R³ für eine Verbindung jeweils einer Zeile der Tabelle A entspricht

### Tabelle 369

Verbindungen der Formel I.6a, in denen (X)ₘ 3-Chlor bedeutet, R² für Ethyl steht und der Rest R³ für eine Verbindung jeweils einer Zeile der Tabelle A entspricht

### Tabelle 370

Verbindungen der Formel I.6a, in denen (X)ₘ 3-Chlor bedeutet, R² für n-Propyl steht und der Rest R³ für eine Verbindung jeweils einer Zeile der Tabelle A entspricht

### Tabelle 371

Verbindungen der Formel I.6a, in denen (X)ₘ 3-Chlor bedeutet, R² für iso-Propyl steht und der Rest R³ für eine Verbindung jeweils einer Zeile der Tabelle A entspricht

### Tabelle 372

Verbindungen der Formel I.6a, in denen (X)ₘ 3-Chlor bedeutet, R² für Cyano steht und der Rest R³ für eine Verbindung jeweils einer Zeile der Tabelle A entspricht

### Tabelle 373

Verbindungen der Formel I.6a, in denen (X)ₘ 4-Chlor bedeutet, R² für Wasserstoff steht und der Rest R³ für eine Verbindung jeweils einer Zeile der Tabelle A entspricht

### Tabelle 374

Verbindungen der Formel I.6a, in denen (X)ₘ 4-Chlor bedeutet, R² für Methyl steht und der Rest R³ für eine Verbindung jeweils einer Zeile der Tabelle A entspricht

### Tabelle 375

Verbindungen der Formel I.6a, in denen (X)ₘ 4-Chlor bedeutet, R² für Ethyl steht und der Rest R³ für eine Verbindung jeweils einer Zeile der Tabelle A entspricht

### Tabelle 376

Verbindungen der Formel I.6a, in denen (X)ₘ 4-Chlor bedeutet, R² für n-Propyl steht und der Rest R³ für eine Verbindung jeweils einer Zeile der Tabelle A entspricht

### Tabelle 377

Verbindungen der Formel I.6a, in denen (X)ₘ 4-Chlor bedeutet, R² für iso-Propyl steht und der Rest R³ für eine Verbindung jeweils einer Zeile der Tabelle A entspricht

### Tabelle 378

Verbindungen der Formel I.6a, in denen (X)ₘ 4-Chlor bedeutet, R² für Cyano steht und der Rest R³ für eine Verbindung jeweils einer Zeile der Tabelle A entspricht

### Tabelle 379

Verbindungen der Formel I.6a, in denen (X)ₘ 5-Chlor bedeutet, R² für Wasserstoff steht und der Rest R³ für eine Verbindung jeweils einer Zeile der Tabelle A entspricht

### Tabelle 380

Verbindungen der Formel I.6a, in denen (X)ₘ 5-Chlor bedeutet, R² für Methyl steht und der Rest R³ für eine Verbindung jeweils einer Zeile der Tabelle A entspricht

### Tabelle 381

Verbindungen der Formel I.6a, in denen (X)ₘ 5-Chlor bedeutet, R² für Ethyl steht und der Rest R³ für eine Verbindung jeweils einer Zeile der Tabelle A entspricht

### Tabelle 382

Verbindungen der Formel I.6a, in denen (X)ₘ 5-Chlor bedeutet, R² für n-Propyl steht und der Rest R³ für eine Verbindung jeweils einer Zeile der Tabelle A entspricht

### Tabelle 383

Verbindungen der Formel I.6a, in denen (X)ₘ 5-Chlor bedeutet, R² für iso-Propyl steht und der Rest R³ für eine Verbindung jeweils einer Zeile der Tabelle A entspricht

### Tabelle 384

Verbindungen der Formel I.6a, in denen (X)ₘ 5-Chlor bedeutet, R² für Cyano steht und der Rest R³ für eine Verbindung jeweils einer Zeile der Tabelle A entspricht

### Tabelle 385

Verbindungen der Formel I.6a, in denen (X)ₘ 3-Brom bedeutet, R² für Wasserstoff steht und der Rest R³ für eine Verbindung jeweils einer Zeile der Tabelle A entspricht

### Tabelle 386

Verbindungen der Formel I.6a, in denen (X)ₘ 3-Brom bedeutet, R² für Methyl steht und der Rest R³ für eine Verbindung jeweils einer Zeile der Tabelle A entspricht

### Tabelle 387

Verbindungen der Formel I.6a, in denen (X)ₘ 3-Brom bedeutet, R² für Ethyl steht und der Rest R³ für eine Verbindung jeweils einer Zeile der Tabelle A entspricht

### Tabelle 388

Verbindungen der Formel I.6a, in denen (X)ₘ 3-Brom bedeutet, R² für n-Propyl steht und der Rest R³ für eine Verbindung jeweils einer Zeile der Tabelle A entspricht

### Tabelle 389

Verbindungen der Formel I.6a, in denen (X)ₘ 3-Brom bedeutet, R² für iso-Propyl steht und der Rest R³ für eine Verbindung jeweils einer Zeile der Tabelle A entspricht

### Tabelle 390

Verbindungen der Formel I.6a, in denen (X)ₘ 3-Brom bedeutet, R² für Cyano steht und der Rest R³ für eine Verbindung jeweils einer Zeile der Tabelle A entspricht

### Tabelle 391

Verbindungen der Formel I.6a, in denen (X)ₘ 4-Brom bedeutet, R² für Wasserstoff steht und der Rest R³ für eine Verbindung jeweils einer Zeile der Tabelle A entspricht

### Tabelle 392

Verbindungen der Formel I.6a, in denen (X)ₘ 4-Brom bedeutet, R² für Methyl steht und der Rest R³ für eine Verbindung jeweils einer Zeile der Tabelle A entspricht

### Tabelle 393

Verbindungen der Formel I.6a, in denen (X)ₘ 4-Brom bedeutet, R² für Ethyl steht und der Rest R³ für eine Verbindung jeweils einer Zeile der Tabelle A entspricht

### Tabelle 394

Verbindungen der Formel I.6a, in denen (X)ₘ 4-Brom bedeutet, R² für n-Propyl steht und der Rest R³ für eine Verbindung jeweils einer Zeile der Tabelle A entspricht

### Tabelle 395

Verbindungen der Formel I.6a, in denen (X)ₘ 4-Brom bedeutet, R² für iso-Propyl steht und der Rest R³ für eine Verbindung jeweils einer Zeile der Tabelle A entspricht

### Tabelle 396

Verbindungen der Formel I.6a, in denen (X)ₘ 4-Brom bedeutet, R² für Cyano steht und der Rest R³ für eine Verbindung jeweils einer Zeile der Tabelle A entspricht

### Tabelle 397

Verbindungen der Formel I.6a, in denen (X)ₘ 5-Brom bedeutet, R² für Wasserstoff steht und der Rest R³ für eine Verbindung jeweils einer Zeile der Tabelle A entspricht

### Tabelle 398

Verbindungen der Formel I.6a, in denen (X)ₘ 5-Brom bedeutet, R² für Methyl steht und der Rest R³ für eine Verbindung jeweils einer Zeile der Tabelle A entspricht

### Tabelle 399

Verbindungen der Formel I.6a, in denen (X)ₘ 5-Brom bedeutet, R² für Ethyl steht und der Rest R³ für eine Verbindung jeweils einer Zeile der Tabelle A entspricht

### Tabelle 400

Verbindungen der Formel I.6a, in denen (X)ₘ 5-Brom bedeutet, R² für n-Propyl steht und der Rest R³ für eine Verbindung jeweils einer Zeile der Tabelle A entspricht

### Tabelle 401

Verbindungen der Formel I.6a, in denen (X)ₘ 5-Brom bedeutet, R² für iso-Propyl steht und der Rest R³ für eine Verbindung jeweils einer Zeile der Tabelle A entspricht

### Tabelle 402

Verbindungen der Formel I.6a, in denen (X)ₘ 5-Brom bedeutet, R² für Cyano steht und der Rest R³ für eine Verbindung jeweils einer Zeile der Tabelle A entspricht

### Tabelle 403

Verbindungen der Formel I.6a, in denen (X)ₘ 3-Nitro bedeutet, R² für Wasserstoff steht und der Rest R³ für eine Verbindung jeweils einer Zeile der Tabelle A entspricht

### Tabelle 404

Verbindungen der Formel I.6a, in denen (X)ₘ 3-Nitro bedeutet, R² für Methyl steht und der Rest R³ für eine Verbindung jeweils einer Zeile der Tabelle A entspricht

### Tabelle 405

Verbindungen der Formel I.6a, in denen (X)ₘ 3-Nitro bedeutet, R² für Ethyl steht und der Rest R³ für eine Verbindung jeweils einer Zeile der Tabelle A entspricht

### Tabelle 406

Verbindungen der Formel I.6a, in denen (X)ₘ 3-Nitro bedeutet, R² für n-Propyl steht und der Rest R³ für eine Verbindung jeweils einer Zeile der Tabelle A entspricht

### Tabelle 407

Verbindungen der Formel I.6a, in denen (X)ₘ 3-Nitro bedeutet, R² für iso-Propyl steht und der Rest R³ für eine Verbindung jeweils einer Zeile der Tabelle A entspricht

### Tabelle 408

Verbindungen der Formel I.6a, in denen (X)ₘ 3-Nitro bedeutet, R² für Cyano steht und der Rest R³ für eine Verbindung jeweils einer Zeile der Tabelle A entspricht

### Tabelle 409

Verbindungen der Formel I.6a, in denen (X)ₘ 4-Nitro bedeutet, R² für Wasserstoff steht und der Rest R³ für eine Verbindung jeweils einer Zeile der Tabelle A entspricht

### Tabelle 410

Verbindungen der Formel I.6a, in denen (X)ₘ 4-Nitro bedeutet, R² für Methyl steht und der Rest R³ für eine Verbindung jeweils einer Zeile der Tabelle A entspricht

### Tabelle 411

Verbindungen der Formel I.6a, in denen (X)ₘ 4-Nitro bedeutet, R² für Ethyl steht und der Rest R³ für eine Verbindung jeweils einer Zeile der Tabelle A entspricht

### Tabelle 412

Verbindungen der Formel I.6a, in denen (X)ₘ 4-Nitro bedeutet, R² für n-Propyl steht und der Rest R³ für eine Verbindung jeweils einer Zeile der Tabelle A entspricht

### Tabelle 413

Verbindungen der Formel I.6a, in denen (X)ₘ 4-Nitro bedeutet, R² für iso-Propyl steht und der Rest R³ für eine Verbindung jeweils einer Zeile der Tabelle A entspricht

### Tabelle 414

Verbindungen der Formel I.6a, in denen (X)ₘ 4-Nitro bedeutet, R² für Cyano steht und der Rest R³ für eine Verbindung jeweils einer Zeile der Tabelle A entspricht

### Tabelle 415

Verbindungen der Formel I.6a, in denen (X)ₘ 5-Nitro bedeutet, R² für Wasserstoff steht und der Rest R³ für eine Verbindung jeweils einer Zeile der Tabelle A entspricht

### Tabelle 416

Verbindungen der Formel I.6a, in denen (X)ₘ 5-Nitro bedeutet, R² für Methyl steht und der Rest R³ für eine Verbindung jeweils einer Zeile der Tabelle A entspricht

### Tabelle 417

Verbindungen der Formel I.6a, in denen (X)ₘ 5-Nitro bedeutet, R² für Ethyl steht und der Rest R³ für eine Verbindung jeweils einer Zeile der Tabelle A entspricht

### Tabelle 418

Verbindungen der Formel I.6a, in denen (X)ₘ 5-Nitro bedeutet, R² für n-Propyl steht und der Rest R³ für eine Verbindung jeweils einer Zeile der Tabelle A entspricht

### Tabelle 419

Verbindungen der Formel I.6a, in denen (X)ₘ 5-Nitro bedeutet, R² für iso-Propyl steht und der Rest R³ für eine Verbindung jeweils einer Zeile der Tabelle A entspricht

### Tabelle 420

Verbindungen der Formel I.6a, in denen (X)ₘ 5-Nitro bedeutet, R² für Cyano steht und der Rest R³ für eine Verbindung jeweils einer Zeile der Tabelle A entspricht

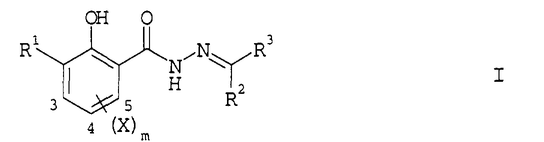

**Tabelle A**

| **Nr.** | **R**^{**3**} | **R**^{**b**} |
|---|---|---|
| A-1 | c-C₆H₁₁ | - |
| A-2 | c-C₆H₁₀ | 2-Cl |
| A-3 | c-C₆H₁₀ | 3-Cl |
| A-4 | c-C₆H₁₀ | 4-Cl |
| A-5 | c-C₆H₁₀ | 2-Br |
| A-6 | c-C₆H₁₀ | 3-Br |
| A-7 | c-C₆H₁₀ | 4-Br |
| A-8 | c-C₆H₁₀ | 2-NO₂ |
| A-9 | c-C₆H₁₀ | 3-NO₂ |
| A-10 | c-C₆H₁₀ | 4-NO₂ |
| A-11 | c-C₆H₁₀ | 2-CN |
| A-12 | c-C₆H₁₀ | 3-CN |
| A-13 | c-C₆H₁₀ | 4-CN |
| A-14 | c-C₆H₁₀ | 2-CH₃ |
| A-15 | c-C₆H₁₀ | 3-CH₃ |
| A-16 | c-C₆H₁₀ | 4-CH₃ |
| A-17 | c-C₆H₉ | 2,4-(Cl)₂ |
| A-18 | c-C₆H₉ | 2,4-(Br)₂ |
| A-19 | c-C₆H₉ | 2,4-(NO₂)₂ |
| A-20 | c-C₆H₉ | 2,4-(CH₃)₂ |
| A-21 | c-C₆H₁₀ | 4-CH₂CH₃ |
| A-22 | c-C₆H₁₀ | 4-CH₂CH₂CH₃ |
| A-23 | c-C₆H₁₀ | 4-CH(CH₃)₂ |
| A-24 | c-C₆H₁₀ | 4-CH₂CH₂CH₂CH₃ |
| A-25 | c-C₆H₁₀ | 4-CH₂CH(CH₃)₂ |
| A-26 | C₆H₅ | - |
| A-27 | C₆H₄ | 2-Cl |
| A-28 | C₆H₄ | 3-Cl |
| A-29 | C₆H₄ | 4-C1 |
| A-30 | C₆H₄ | 2-Br |
| A-31 | C₆H₄ | 3-Br |
| A-32 | C₆H₄ | 4-Br |
| A-33 | C₆H₄ | 2-NO₂ |
| A-34 | C₆H₄ | 3-NO₂ |
| A-35 | C₆H₄ | 4-NO₂ |
| A-36 | C₆H₄ | 2-CN |
| A-37 | C₆H₄ | 3-CN |
| A-38 | C₆H₄ | 4-CN |
| A-39 | C₆H₄ | 2-CH₃ |
| A-40 | C₆H₄ | 3-CH₃ |
| A-41 | C₆H₄ | 4-CH₃ |
| A-42 | C₆H₄ | 4-CH₂CH₃ |
| A-43 | C₆H₄ | 4-CH₂CH₂CH₃ |
| A-44 | C₆H₄ | 4-CH(CH₃)₂ |
| A-45 | C₆H₄ | 4-CH₂CH₂CH₂CH₃ |
| A-46 | C₆H₄ | 4-CH₂CH(CH₃)₂ |
| A-47 | C₆H₄ | 4-C(CH₃)₃ |
| A-48 | C₆H₄ | 4-C₆H₅ |
| A-49 | C₆H₄ | 4-(2-Cl)-C₆H₄ |
| A-50 | C₆H₄ | 4-(3-Cl)-C₆H₄ |
| A-51 | C₆H₄ | 4-(4-Cl)-C₆H₄ |
| A-52 | C₆H₄ | 4-(2-Br)-C₆H₄ |
| A-53 | C₆H₄ | 4-(3-Br)-C₆H₄ |
| A-54 | C₆H₄ | 4-(4-Br)-C₆H₄ |
| A-55 | C₆H₄ | 4-(2-NO₂)-C₆H₄ |
| A-56 | C₆H₄ | 4-(3-NO₂)-C₆H₄ |
| A-57 | C₆H₄ | 4-(4-NO₂)-C₆H₄ |
| A-58 | C₆H₄ | 4-(2-CN)-C₆H₄ |
| A-59 | C₆H₄ | 4-(3-CN)-C₆H₄ |
| A-60 | C₆H₄ | 4-(4-CN)-C₆H₄ |
| A-61 | C₆H₄ | 4-(2-CH₃)-C₆H₄ |
| A-62 | C₆H₄ | 4-(3-CH₃)-C₆H₄ |
| A-63 | C₆H₄ | 4-(4-CH₃)-C₆H₄ |
| A-64 | C₆H₄ | 4-(2,4-Cl₂)-C₆H₃ |
| A-65 | C₆H₄ | 4-(2,4-Br₂)-C₆H₃ |
| A-66 | C₆H₄ | 4-[2,4-(NO₂)₂]-C₆H₃ |
| A-67 | C₆H₄ | 4-[2,4-(CH₃)₂]-C₆H₃ |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten*, *Phycomyceten* und *Basidiomyceten*, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.- Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS, 3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yl-oxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1: Herstellung von 2-Hydroxy-3-nitrobenzoesäurehydrazid

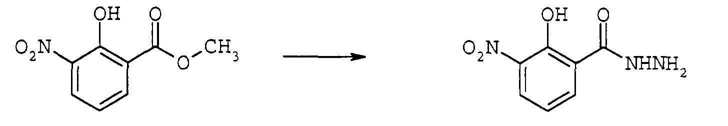

Zu einer Lösung von 30 g (0,15 mol) 3-Nitrosalicylsäuremethylester in 200 ml wasserfr. Tetrahydrofuran (THF) wurden 40 ml Hydrazinhydrat gegeben. Nach Verdünnen mit THF war die entstandene Suspension rührbar, sie wurde etwa 18 Std. refluxiert. Nach dem Abkühlen wurde auf Eis gegeben. Die wässrige Lösung wurde durch Zugabe von NaOH-Lösung neutralisiert. Es fielen Kristalle aus, die abfiltriert und mit Wasser gewaschen wurden. Nach dem Trocknen erhielt man 27 g (90%) der Titelverbindung.

### Beispiel 2: Herstellung von 3-Nitrosalicylsäure-(1-phenylethyliden)-hydrazid

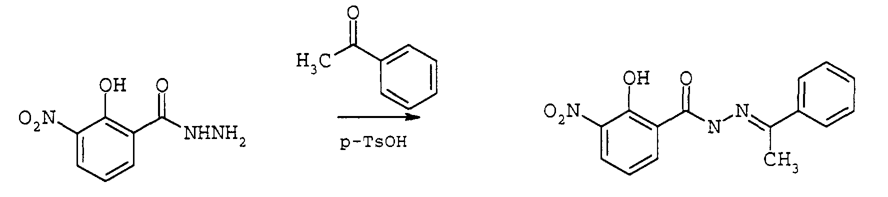

Eine Lösung von 1,0 g (5 mmol) 3-Nitrosalicylsäurehydrazid wurden in 50 ml wasserfr. Ethanol mit 0,6 g (5 mmol) Acetophenon, sowie einer katalytischen Menge p-Toluolsulfonsäure versetzt. Nach 14 Std. Rühren bei etwa 20 bis 25°C und Abdestillieren des Lösungsmittels erherhielt man 1,4 g der Titelverbindung (90% d. Th.) als farblose Kristalle vom Fp.: 163-168°C.

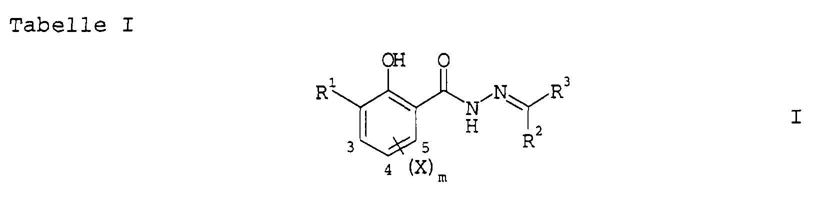

| **Nr.** | **R**^{**1**} | **(X)**_{**m**} | **R**^{**2**} | **R**^{**3**} | **Schmp. [°C]** |
|---|---|---|---|---|---|
| I-1 | NO₂ | H | CH₃ | C₆H₅ | 163-168 |
| I-2 | NO₂ | H | H | C₆H₅ | 193-199 |
| I-3 | NO₂ | H | CH₃ | 4-CH₃-C₆H₄ | 149-154 |
| I-4 | NO₂ | H | H | 4-CH₃-C₆H₄ | 160-166 |
| I-5 | NO₂ | H | CH₃ | 4-Cl-C₆H₄ | 194-197 |
| I-6 | NO₂ | H | H | 4-Cl-C₆H₄ | 192-196 |
| I-7 | NO₂ | H | CH₃ | 4-Br-C₆H₄ | 194-197 |
| I-8 | NO₂ | H | H | 4-Br-C₆H₄ | 174-178 |
| I-9 | NO₂ | H | CH₃ | 4-NO₂-C₆H₄ | 204-208 |
| I-10 | NO₂ | H | H | 4-NO₂-C₆H₄ | 240-244 |
| I-11 | NO₂ | H | CH₃ | 4-CN-C₆H₄ | 218-222 |
| I-12 | NO₂ | H | H | 4-CN-C₆H₄ | 220-223 |
| I-13 | NO₂ | H | CH₃ | 4-OCH₃-C₆H₄ | 146-150 |
| I-14 | NO₂ | H | H | 4-OCH₃-C₆H₄ | 167-172 |
| I-15 | NO₂ | H | CH₃ | 2,4-(OCH₃)₂-C₆H₄ | 245-248 |
| I-16 | NO₂ | H | H | 2,4-(OCH₃)₂-C₆H₄ | 207-210 |
| I-17 | NO₂ | H | CH₃ | 2,4-Cl₂-C₆H₄ | 110-114 |
| I-18 | NO₂ | H | H | 2,4-Cl₂-C₆H₄ | 219-223 |
| I-19 | NO₂ | H | CH₃ | 4-C₆H₅-C₆H₄ | 209-212 |
| I-20 | NO₂ | H | H | 4-C₆H₅-C₆H₄ | 189-195 |

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel - Wirksamkeit gegen Botrytis cinerea an Paprikablättern

Paprikasämlinge der Sorte "Neusiedler Ideal Elite" wurden, nachdem sich 4 - 5 Blätter gut entwickelt hatten, mit einer wäßrigen Wirkstoffaufbereitung, die aus einer Stammlösung aus 10 % Wirkstoff, 63 % Cyclohexanon und 27 % Emulgiermittel angesetzt wurde, bis zur Tropfnässe besprüht. Am nächsten Tag wurden die behandelten Pflanzen mit einer Sporensuspension von Botrytis cinerea, die 1.7 x 10⁶ Sporen/ml in einer 2 %igen wäßrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefall auf den Blättern visuell in % ermittelt werden.

In diesem Test zeigten die mit 250ppm-haltiger Aufbereitung der Wirkstoffe I-1, I-3 und I-6 behandelten Pflanzen maximal 15 % Befall, während die unbehandelten Pflanzen zu 90 % befallen waren.

## Patentansprüche

1. Salicylsäurehydrazid-Derivate der Formel I, 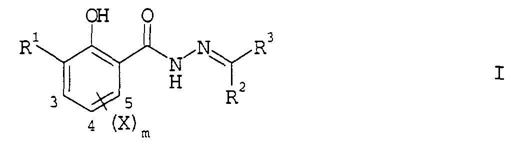 in der der Index und die Substituenten folgende Bedeutung haben:
X Halogen, NO₂, Cyano, C₁-C₄-Alkyl oder C₁-C₄-Alkoxy;
m 0, 1, 2 oder 3, wobei die Substituenten X verschieden sein können, wenn n größer als 1 ist;
R¹ NO₂, NH₂ oder NH-CO-A;
A Wasserstoff, C₁-C₄-Alkyl, C₁-C₄-Alkoxy, NH₂, NHCH₃ oder N(CH₃)₂;
R² Wasserstoff, Cyano, C₁-C₆-Alkyl, C₂-C₆-Alkenyl, C₂-C₆-Alkinyl, C₁-C₆-Halogenalkyl, C₁-C₆-Alkoxy oder C₁-C₆-Alkylthio;
wobei die Kohlenwasserstoffreste unsubstituiert oder partiell oder vollständig halogeniert sind oder eine bis drei Gruppen R^{a} tragen können
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino,
Di-C₁-C₆-alkylamino, C₂-C₆-Alkenyl, C₃-C₆-Alkenyloxy, C₃-C₆-Alkinyloxy oder C₁-C₄-Alkylendioxy, welches halogeniert sein kann,
R³ Phenyl, Naphthyl, C₃-C₁₀-Cycloalkyl, 5-gliedriges oder 6-gliedriges Hetaryl oder 5-gliedriges oder 6-gliedriges Heterocyclyl, enthaltend ein bis drei N-Atome und/oder ein O- oder S-Atom oder. ein oder zwei O- und/oder S-Atome, wobei die Ringsysteme unsubstituiert oder substituiert sind durch einen bis drei Reste R^{b}:
R^{b} Cyano, Nitro, Amino, Aminocarbonyl, Aminothiocarbonyl, Halogen, Hydroxy, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Phenyl, Phenoxy, Benzyl, Benzyloxy, 5- oder 6-gliedriges Heterocyclyl, 5- oder 6-gliedriges Hetaryl, 5- oder 6-gliedriges Hetaryloxy, C(=NOR^{α})-OR^{β} oder OC (R^{α})₂-C(R^{β})=NOR^{β},
wobei die cyclischen Reste ihrerseits unsubstituiert oder substituiert sind durch einen bis drei Reste R^{c}:
R^{c} Cyano, Nitro, Halogen, Hydroxy, Amino, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkenyl, Phenyl, Phenoxy, Phenylthio, Benzyl, Benzyloxy,
5- oder 6-gliedriges Heterocyclyl, 5- oder 6-gliedriges Hetaryl, 5- oder 6-gliedriges Hetaryloxy oder C(=NOR^{α})-OR^{β};
R^{α}, R^{β} Wasserstoff oder C₁-C₆-Alkyl.

2. Verbindungen der Formel I nach Anspruch 1, in der R¹ für NH-CO-A steht.

3. Verbindungen der Formel I nach Ansprüchen 1 oder 2, in der m Null bedeutet.

4. Verbindungen der Formel I nach Ansprüchen 1 bis 3, in der m 1 bedeutet und X in 4-Stellung steht.

5. Verbindungen der Formel I nach Ansprüchen 1 bis 4, wobei
A Wasserstoff, C₁-C₄-Alkoxy, NHCH₃ oder N(CH₃)₂;
R² CN oder C₁-C₆-Alkyl;
R³ Phenyl oder 5-gliedriges oder 6-gliedriges Hetaryl, wobei die Ringsysteme unsubstituiert oder substituiert sind durch einen bis drei Reste R^{b};
bedeuten.

6. Verfahren zur Herstellung von Verbindungen der Formel I nach Anspruch 1, durch Umsetzung von Hydraziden der Formel II 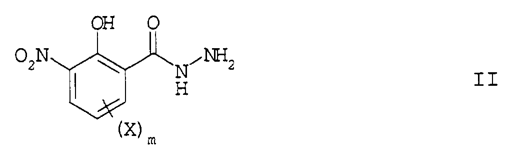 mit Carbonylverbindungen der Formel III 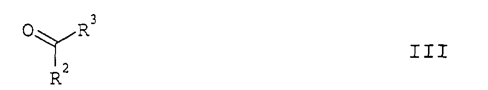 zu Verbindungen der Formel I, in der R¹ NO₂ bedeutet, 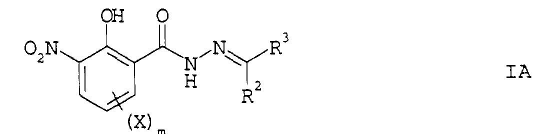 und, zur Herstellung von Verbindungen I, in denen R¹ NH₂ oder NH-CO-A bedeutet, Hydrierung von IA zu Aminophenolverbindungen der Formel IB, 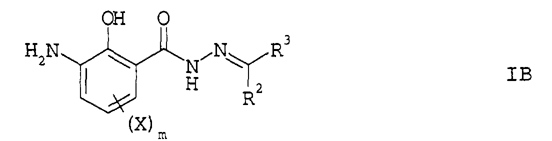 und, zur Herstellung von Verbindungen I, in denen R¹ NH-CO-H bedeutet, Formylierung von IB zu Verbindungen der Formel I.1 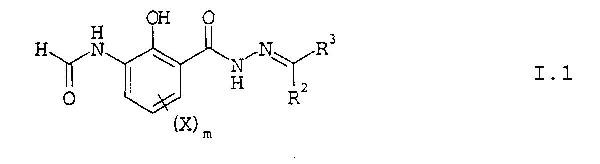 oder, zur Herstellung von Verbindungen I, in denen R¹ für NH-CO-NH₂ steht, Umsetzung von IB mit Alkali- oder Erdalkaliisocyanaten zu Verbindungen der Formel I.2, 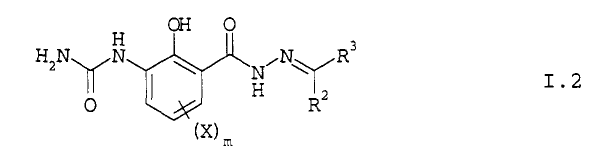 oder, zur Herstellung von Verbindungen I, in denen R¹ für NH-CO-NHCH₃ steht, Umsetzung von IB mit Methylisocyanat zu Verbindungen der Formel I.3, 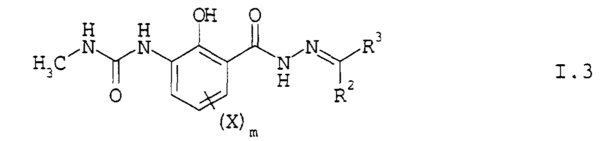 oder, zur Herstellung von Verbindungen I, in denen R¹ für NH-CO-N(CH₃)₂ steht, Umsetzung von IB mit Phosgen und Dimethylamin zu Verbindungen der Formel I.4, 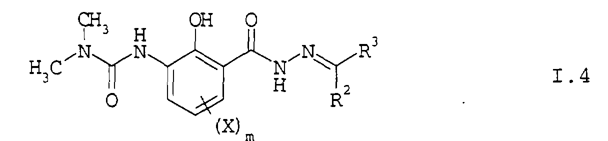 oder, zur Herstellung von Verbindungen I, in denen R¹ für NH-CO-OCH₃ steht, Umsetzung von IB mit Kohlenmonoxid und Methanol unter Übergangsmetallkatalyse zu Verbindungen der Formel I.5 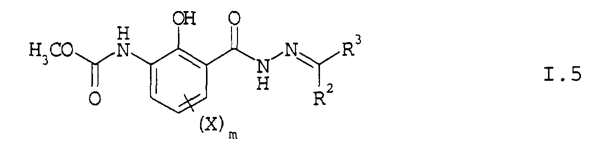 umgesetzt werden.

7. Verwendung der Verbindungen der Formeln IA und IB als Zwischenprodukte.

8. Zur Bekämpfung von pflanzenpathogenen Schadpilzen geeignetes Mittel, enthaltend einen festen oder flüssigen Trägerstoff und eine Verbindung der Formel I gemäß Anspruch 1.

9. Verfahren zur Bekämpfung von pflanzenpathogenen Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.
